# EUROPÄISCHE PATENTSCHRIFT

(11) **EP 3 083 763 B1**
(45) Veröffentlichungstag und Bekanntmachung des Hinweises auf die Patenterteilung: **07.02.2018**
(21) Anmeldenummer: 14821110.5
(22) Anmeldetag: 15.12.2014
(51) Int. Cl.: C08G 77/14, C08L 83/04

(54) **GLYCOSIDRESTE AUFWEISENDE ORGANOPOLYSILOXANGELE**
POLYORGANOSILOXANE GELS HAVING GLYCOSIDE GROUPS
GELS D'ORGANOPOLYSILOXANES CONTENANT DES RESTES GLYCOSIDIQUES

(30) Priorität: 17.12.2013 DE 102013226252
(43) Veröffentlichungstag der Anmeldung: 26.10.2016
(73) Patentinhaber: Wacker Chemie AG, 81737 München (DE)
(72) Erfinder: KNÖR, Sebastian, 84547 Emmerting (DE)
(74) Vertreter: Deffner-Lehner, Maria
(86) Internationale Anmeldenummer: PCT/EP2014/077797
(87) Internationale Veröffentlichungsnummer: WO 2015/091378

(56) Entgegenhaltungen:
- EP-A1- 1 132 430
- DE-T2-602005 002 262
- US-A- 5 831 080

## Beschreibung

Die Erfindung betrifft Glycosidreste aufweisende Organopolysiloxangele, Verfahren zu deren Herstellung, sowie deren Anwendung in kosmetischen Formulierungen.

Organopolysiloxangele können durch Vernetzung eines ungesättigten Organopolysiloxanharzes mit einem Si-H-haltigen Organopolysiloxan, im Weiteren auch Si-H-funktioneller Vernetzer genannt, in Gegenwart eines Verdünnungsmittels hergestellt werden.

Vernetzungen sind Verbindungen von Polymerketten in einem dreidimensionalen Netzwerk. Sie können als langkettige Verzweigungen betrachtet werden, die so zahlreich sind, dass ein kontinuierliches unlösliches Netzwerk oder Gel gebildet wird.

Organopolysiloxannetzwerke werden häufig über platinkatalysierte Hydrosilylierungsreaktionen hergestellt. Dabei werden häufig ein Si-H-haltiges Organopolysiloxan und ein Vinyl-funktionelles Organopolysiloxan miteinander zur Reaktion gebracht. Eine wesentliche Voraussetzung für den Aufbau eines 3-dimensionalen Netzwerkes ist dabei, dass mindestens eine der beiden Komponenten, das Si-H-haltige Organopolysiloxan oder das Vinyl-funktionelles Organopolysiloxan, in der mittleren Zusammensetzung mehr als zwei Funktionalitäten pro Molekül aufweist.

Die platinkatalysierte Hydrosilylierungsreaktion bietet bei der Ausbildung von Organopolysiloxannetzwerken den Vorteil, dass keine Nebenprodukte gebildet werden, und dass Verknüpfungsstellen und Netzwerkarchitektur eng definiert sind.

Der wichtigste Grund für die Anwendung von Organopolysiloxangelen in kosmetischen Anwendungen sind die dadurch erzielten sensorischen Vorteile, insbesondere die Verbesserung des Hautgefühls von kosmetischen Formulierungen. Darüber hinaus dienen Organopolysiloxangele als Verdickungsmittel in kosmetischen Formulierungen.

US 6,423,322 B1 und WO 2013/156390 A1 offenbaren Organopolysiloxangele, die durch Hydrosilylierungsreaktion eines speziellen, vinylfunktionellen MQ-Harzes mit einem Si-H-haltigen Organopolysiloxan in Gegenwart eines Verdünnungsmittels und einer kleinen Menge Platin-Hydrosilylierungskatalysator leicht hergestellt werden können. Die resultierenden Gele ziehen keine Fäden und lassen sich leicht zu einer stabilen Creme oder Paste homogenisieren. Nachteilig an solchen Gele ist jedoch, dass Sie nur eine geringe Verträglichkeit mit polaren organischen Substanzen, Alkoholen oder Wasser aufweisen. Dadurch sind solche Gele auch nicht in der Lage, wichtige Kosmetik-Inhaltsstoffe, wie Wasser oder Glycerin in hinreichenden Mengen aufzunehmen und zeigen in wässrigen oder alkoholischen Mischungen keine verdickende Wirkung. Dadurch eignen sich solche Gele auch nicht oder nur eingeschränkt für die Herstellung von wasser-basierten oder alkohol-basierten Kosmetikprodukten.

US 2010/0330011 A1 offenbart Organopolysiloxangele, die durch Vernetzung eins Si-H-haltigen Organopolysiloxans mit einem beidseitig ungesättigten Polyoxyalkylen in einem Verdünnungsmittel hergestellt werden. Die Verwendung von Polyoxyalkylenen und deren Derivate in kosmetischen Produkten ist mit großen Nachteilen versehen. Sie werden aus hochgiftigen Rohstoffen gefertigt und ihre Verwendung in kosmetischen Produkten ist sehr umstritten, da sie teilweise im Verdacht stehen eine irritierende und sensibilisierende Wirkung zu haben und auch die Haut durchlässig für andere Giftstoffe machen können.

EP 1 132 430 A1 offenbart Organopolysiloxangele, die durch Hydrosilylierungsreaktion eines speziellen, vinylfunktionellen MQ-Harzes und eines polyoxyethylierten oder polyoxypropylierten Allylalkohols mit einem hoch Si-H-haltigen Organopolysiloxan mit ungefähr 0,5 Gew.% siliciumgebundenen Wasserstoffatomen in Gegenwart von Decamethylcyclopentasiloxan als Verdünnungsmittel und einer kleinen Menge Platin-Hydrosilylierungskatalysator hergestellt werden können. Die resultierenden Gele ziehen keine Fäden und lassen sich zu einer stabilen Creme oder Paste homogenisieren. Die Verwendung eines hoch Si-H-haltigen Vernetzers mit 0,54 Gew.-% Si-gebundenen Wasserstoff wird als besonders bevorzugt beschrieben. Die Verwendung eines weniger funktionellen Vernetzers in untergeordneten Mengen soll möglich sein.
Ein wesentlicher Nachteil dieser Organopolysiloxangele ist jedoch insbesondere, dass das erzeugte Hautgefühl für kosmetische Anwendungen nicht ideal ist. Aufgrund des relativ hohen Anteils an vinylfunktionellen MQ-Harz sind solche Gele auch vergleichsweise teuer herzustellen. Darüber hinaus zeigt sich, dass keine geeigneten Gele hergestellt werden können, wenn lineare Organopolysiloxane als Verdünnungsmittel verwendet werden. Lineare Organopolysiloxane erlangen jedoch zunehmend Bedeutung in kosmetischen Formulierungen, nachdem cyclische Organopolysiloxane, wie in den in EP 1 132 430 A1 offenbarten Organopolysiloxangelen verwendet, aufgrund ihrer möglicherweise toxischen Wirkung zunehmend in kosmetischen Formulierungen gemieden werden.

Es bestand die Aufgabe, Organopolysiloxangele mit verbesserten Eigenschaften, insbesondere mit einem verbesserten Hautgefühl, bereitzustellen, welche die oben genannten Nachteile nicht aufweisen. Die Aufgabe wird durch die Erfindung gelöst.

Gegenstand der Erfindung sind Glycosidreste aufweisende Organopolysiloxangele hergestellt durch Umsetzung von
(1a) ungesättigten Organopolysiloxanharzen und
(1b) Verbindungen, die einen Glycosidrest und eine hydrosilylierbare Endgruppe aufweisen, mit der Maßgabe, dass der gewichtsmäßige Anteil, bezogen auf das Gesamtgewicht des Organopolysiloxangels, 0,1 bis 10 Gew.-%, vorzugsweise 0,1 bis 5 Gew-%, bevorzugt 0,3 bis 2 Gew.-%, ist,
   mit
(2) Si-H funktionellen Organopolysiloxanen der allgemeinen Formel

   H_{c}R_{3-c}SiO(R₂SiO)ₐ(RHSiO)_{b}SiR_{3-c}H_{c} (I),

   wobei
   c 0 oder 1, vorzugsweise 0, ist,
   R gleich oder verschieden sein kann und einen einwertigen, gegebenenfalls substituierten Kohlenwasserstoffrest mit 1 bis 18 Kohlenstoffatomen je Rest bedeutet,
   a und b ganze Zahlen sind, mit der Maßgabe, dass die Summe a+b 66 bis 248, vorzugsweise 98 bis 248, bevorzugt 118 bis 168 ist,
   dass die Organopolysiloxane (2) Si-gebundenen Wasserstoff in Mengen von 0,011 bis 0,044 Gew.-%, vorzugsweise von 0,019 bis 0,044 Gew.-%, bevorzugt von 0,022 bis 0,032 Gew.-%, enthalten,
   und dass die Anzahl an Si-H-Gruppen pro Molekül in der durchschnittlichen Zusammensetzung größer 2 und kleiner als 5 ist,
   oder Mischungen von (2) Si-H funktionellen Organopolysiloxanen mit
(2') Si-H funktionellen Organopolysiloxanen der allgemeinen Formel

   H_{c'}R_{3-c'}SiO(R₂SiO)_{a'}(RHSiO)_{b'}SiR_{3-c'}H_{c'} (I'),

   wobei
   c' 0 oder 1, vorzugsweise 0 ist,
   R die oben dafür angegebene Bedeutung hat,
   a' und b' ganze Zahlen sind, mit der Maßgabe, dass die Summe a'+b' 8 bis 248, vorzugsweise 38 bis 248, ist, und dass die Organopolysiloxane (2') Si-gebundenen Wasserstoff in Mengen von 0,045 bis 0,35 Gew.-%, vorzugsweise von 0,045 bis 0,156 Gew.-%, enthalten,
   in Gegenwart von
(3) die Anlagerung von Si-gebundenem Wasserstoff an aliphatische Mehrfachbindung fördernde Katalysatoren,
   wobei (1a), (1b) und (2) oder Mischungen von (2) und (2') in
(4) Verdünnungsmitteln, vorzugsweise Organopolysiloxanen mit 2 bis 200 Si-Atomen, bevorzugt Organopolysiloxanen mit 2 bis 50 Si-Atomen, besonders bevorzugt lineare Organopolysiloxanen mit einer Viskosität von 1,5 bis 50 mm2/s bei 25°C, oder organischen Verdünnungsmitteln oder Mischungen von Organopolysiloxanen mit 2 bis 200 Si-Atomen und organischen Verdünnungsmitteln,
   dispergiert sind.

Gegenstand der Erfindung ist weiterhin ein Verfahren zur Herstellung der erfindungsgemäßen Organopolysiloxangele indem
(1a) ungesättigte Organopolysiloxanharze und
(1b) Verbindungen, die einen Glycosidrest und eine hydrosilylierbare Endgruppe aufweisen,
   mit der Maßgabe, dass der gewichtsmäßige Anteil, bezogen auf das Gesamtgewicht der Organopolysiloxangele, 0,1 bis 10 Gew.-%, bevorzugt 0,1 bis 5 Gew-%, besonders bevorzugt 0,3 bis 2 Gew.-%, ist, mit
(2) Si-H funktionellen Organopolysiloxanen der allgemeinen Formel

   H_{c}R_{3-c}SiO(R₂SiO)ₐ(RHSiO)_{b}SiR_{3-c}H_{c} (I),

   wobei
   c 0 oder 1, vorzugsweise 0, ist,
   R gleich oder verschieden sein kann und einen einwertigen, gegebenenfalls substituierten Kohlenwasserstoffrest mit 1 bis 18 Kohlenstoffatomen je Rest bedeutet,
   a und b ganze Zahlen sind, mit der Maßgabe, dass die Summe a+b 66 bis 248, vorzugsweise 98 bis 248, bevorzugt 118 bis 168 ist,
   dass die Organopolysiloxane (2) Si-gebundenen Wasserstoff in Mengen von 0,011 bis 0,044 Gew.-%, vorzugsweise von 0,019 bis 0,044 Gew.-%, bevorzugt von 0,022 bis 0,032 Gew.-%, enthalten,
   und dass die Anzahl an Si-H-Gruppen pro Molekül in der durchschnittlichen Zusammensetzung größer 2 und kleiner als 5 ist,
   oder Mischungen von (2) Si-H funktionellen Organopolysiloxanen mit
(2') Si-H funktionellen Organopolysiloxanen der allgemeinen Formel

   H_{c}R_{3-c}SiO(R₂SiO)ₐ(RHSiO)_{b}SiR_{3-c}H_{c} (I'),

   wobei
   c 0 oder 1, vorzugsweise 0 ist,
   R die oben dafür angegebene Bedeutung hat,
   a und b ganze Zahlen sind, mit der Maßgabe, dass
      die Summe a+b 8 bis 248, vorzugsweise 38 bis 248, ist,
      und dass die Organopolysiloxane (2') Si-gebundenen Wasserstoff in Mengen von 0,045 bis 0,35 Gew.-%, vorzugsweise von 0,045 bis 0,156 Gew.-%, enthalten,
   in Gegenwart von
(3) die Anlagerung von Si-gebundenem Wasserstoff an aliphatische Mehrfachbindung fördernde Katalysatoren, umgesetzt werden,
   wobei (1a), (1b) und (2) oder Mischungen von (2) und (2') in
(4) Verdünnungsmitteln, bevorzugt Organopolysiloxanen mit 2 bis 200 Si-Atomen, bevorzugt Organopolysiloxanen mit 2 bis 50 Si-Atomen, besonders bevorzugt lineare Organopolysiloxanen mit einer Viskosität von 1,5 bis 50 mm²/s bei 25°C, oder organischen Verdünnungsmitteln oder Mischungen von Organopolysiloxanen mit 2 bis 200 Si-Atomen und organischen Verdünnungsmitteln,
dispergiert sind.

Werden bei der Herstellung der erfindungsgemäßen Organopolysiloxangele Mischungen von Si-H funktionellen Organopolysiloxanen (2) und (2') eingesetzt, ist das GewichtsVerhältnis von (2) zu (2') vorzugsweise größer 0,2, bevorzugt größer 0,5, besonders bevorzugt größer 1.

Völlig überraschend wurde gefunden, dass gerade dann besonders vorteilhafte Gele erhalten werden, wenn das Organopolysiloxangel unter Verwendung eines Si-H-haltigen Vernetzers mit besonders niedrigerem Si-H-Gehalt hergestellt wird und ein Glycosidrest kovalent an das Organopolysiloxangel gebunden ist. Die erfindungsgemäßen Gele weisen ein exzellentes Hautgefühl auf. Dieses Ergebnis ist gerade deswegen so unerwartet, weil man Zucker im allgemeine eher mit einem klebrigen oder auch einem stärkenden Effekt verbindet. Darüber hinaus besitzen die Gele eine verbesserte Kompatibilität mit polaren organischen Substanzen und sind sogar in der Lage, äußerst hydrophile Flüssigkeiten, wie Wasser oder Glycerin, aufzunehmen, ohne die viskose Gelstruktur zu verlieren.

Im Rahmen dieser Erfindung sollen die Formeln (I) und (I') so verstanden werden, dass a Einheiten -(R₂SiO)- und b Einheiten-(RHSiO)- in beliebiger Weise im Organopolysiloxanmolekül verteilt sein können.

Die erfindungsgemäß eingesetzten Si-H-haltigen Organopolysiloxane (2) haben vorzugsweise eine Viskosität von 50 bis 2000 mm²/s, bevorzugt 100 bis 1000 mm²/s, besonders bevorzugt 150 bis 600 mm²/s, jeweils bei 25°C, und ein molares Verhältnis a:(b+c) von vorzugsweise 30:1 bis 150:1, bevorzugt 30:1 bis 80:1, besonders bevorzugt 40:1 bis 70:1. Die in den Mischungen mit (2) eingesetzten Si-H-haltigen Organopolysiloxane (2') haben vorzugsweise eine Viskosität von 3 bis 2000 mm²/s, besonders bevorzugt 20 bis 1200 mm²/s, jeweils bei 25°C, und ein molares Verhältnis a:(b+c) von vorzugsweise 4:1 bis 30:1, besonders bevorzugt 8:1 bis 30:1.

Überraschenderweise wurde gefunden, dass die erfindungsgemäßen Organopolysiloxangele auf Basis der Si-H-haltigen Organopolysiloxane (2) oder auf Basis der Mischung von (2) und (2') wesentlich bessere sensorische Eigenschaften, insbesondere ein besseres Hautgefühl aufweisen, als Gele auf Basis eines Organopolysiloxans mit relativ hohen Gehalt an siliciumgebundenen Wasserstoffatomen, wie sie in EP 1 132 430 A1 offenbart sind. Sie sind außergewöhnlich gleitfähig und fühlen sich nicht unerwünscht ölig an. Nach dem Verteilen auf der Haut hinterlassen sie ein geschmeidigeres Hautgefühl und lassen kein unerwünscht öliges, filmartiges oder stumpfes Gefühl zurück.

Beispiele für Reste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl und Methylcyclohexylreste; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der α- und der β-Phenylethylrest.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorphenylrest.

Bevorzugt handelt es sich bei dem Rest R um einen einwertigen Kohlenwasserstoffrest mit 1 bis 6 Kohlenstoffatomen, wobei der Methylrest besonders bevorzugt ist.

Bei den in den erfindungsgemäßen Organopolysiloxangelen eingesetzten ungesättigten Organopolysiloxanharzen (1a) handelt es sich vorzugsweise um ungesättigte Organopolysiloxanharze aufgebaut aus Einheiten der allgemeinen Formel (II)

RₓR'_{y}SiO_{(4-x-y)/2} (II)

wobei
R die oben dafür angegebene Bedeutung hat,
R' einen einwertigen Kohlenwasserstoffrest bedeutet, an den Si-H-Gruppen in einer Hydrosilylierungsreaktion angelagert werden können, vorzugsweise einen einwertigen eine terminale, aliphatische C-C-Mehrfachbindung aufweisenden Kohlenwasserstoffrest mit 2 bis 18 Kohlenstoffatomen, bevorzugt einen ω-Alkenylrest mit 2 bis 12 C-Atomen, besonders bevorzugt einen Vinylrest bedeutet,
x 0, 1, 2 oder 3,
y 0, 1 oder 2, vorzugsweise 0 oder 1, ist,
mit der Maßgabe, dass die Summe x+y kleiner gleich 3 ist, und dass pro Molekül mindestens 2 Reste R', vorzugsweise mindestens 3 Reste R', mindestens 20 Mol-% T- und/oder Q-Einheiten (T-Einheiten: Summe x+y=1; Q-Einheiten: Summe x+y=0), vorzugsweise mindestens 20 Mol-% Q-Einheiten, vorhanden sein müssen, und daneben D-Einheiten (Summe x+y=2) vorhanden sein können.

Bevorzugt handelt es sich bei den ungesättigten Organopolysiloxanharzen der Formel (II) um MQ-Harze aus Einheiten der Formeln

SiO₂ (Q-Einheiten)

und

R₃SiO_{1/2} und R₂R'SiO_{1/2} (M-Einheiten)

wobei R und R' die oben dafür angegebene Bedeutung haben.
Das molare Verhältnis von M- zu Q-Einheiten liegt dabei vorzugsweise im Bereich von 0,5 bis 4,0, bevorzugt im Bereich von 0,5 bis 2,0, besonders bevorzugt im Bereich von 0,6 bis 1,5. Diese Siliconharze können außerdem bis zu 10 Gew.-% freie Hydroxy- oder Alkoxygruppen enthalten.

Vorzugsweise haben die ungesättigten Organopolysiloxanharze (1a) bei 25°C eine Viskosität größer als 0,7 mm²/s, besonders bevorzugt sind solche Harze, die bei 25 °C eine Viskosität von größer als 1000 mm²/s haben oder Feststoffe sind. Das mit Gelpermeationschromatografie bestimmte gewichtsmittlere Molekulargewicht M_{w} (bezogen auf einen Polystyrolstandard) dieser Harze beträgt vorzugsweise 334 bis 200000 g/mol, bevorzugt 1000 bis 20000 g/mol.

Die ungesättigten Organopolysiloxanharze (1a) der erfindungsgemäßen Organopolysiloxangele haben vorzugsweise eine Jodzahl kleiner 254 und bevorzugt sind Organopolysiloxanharze mit einer Jodzahl kleiner 76.

Der ungesättigte Kohlenwasserstoffrest ist vorzugsweise gebunden an eine M-Einheit (=M_{Vi}) oder D-Einheit (=D_{Vi}), bevorzugt an eine M-Einheit, wobei das molare Verhältnis M: (M_{Vi}+D_{Vi}), vorzugsweise M:M_{Vi}, vorzugsweise im Bereich 0 bis 50, bevorzugt im Bereich 0 bis 20, besonders bevorzugt im Bereich 2,5 bis 13 liegt.

Beispiele für Reste R' sind Alkenylreste, wie der Vinyl-, 5-Hexenyl-, Cyclohexenyl-, 1-Propenyl-, Allyl-, 3-Butenyl- und 4-Pentenylrest, und Alkinylreste, wie der Ethinyl-, Propargyl- und 1-Propinylrest. Bevorzugt handelt es sich bei dem Rest R' um Alkenylreste, besonders bevorzugt ω-Alkenylreste, insbesondere um den Vinylrest.

Erfindungsgemäß eingesetzte Verbindungen (1b), die einen Glycosidrest und eine hydrosilylierbare Endgruppe aufweisen, sind vorzugsweise solche der allgemeinen Formel

Z-(R¹O)ₚ-R² (III),

wobei
Z einen Glycosidrest bedeutet, der aus 1 bis 10 Monosaccharideinheiten, vorzugsweise Glucoseeinheiten, aufgebaut ist,
R¹ gleich oder verschieden sein kann und einen zweiwertigen Kohlenwasserstoffrest mit 2 bis 4 C-Atomen bedeutet,
R² einen einwertigen Kohlenwasserstoffrest mit 2 bis 12 C-Atomen, an den Si-H-Gruppen in einer Hydrosilylierungsreaktion angelagert werden können, vorzugsweise einen einwertigen eine terminale aliphatische C-C-Mehrfachbindung aufweisenden Kohlenwasserstoffrest mit 2 bis 12 C-Atomen, bevorzugt 3 bis 12 C-Atomen, besonders bevorzugt einen Allylrest, bedeutet, und
p 0 oder eine ganze Zahl von 1 bis 20 ist.

) Beispiele für Verbindungen (1b) sind

G-CH₂CH=CH₂,

G- (CH₂CH₂O) -CH₂CH=CH₂,

G₂-CH₂CH=CH₂,

G₂- (CH₂CH₂O) -CH₂CH=CH₂,

wobei G einen Glycosidrest der Formel (C₆H₁₁O₆) - und
G₂ einen aus zwei Glucoseeinheiten aufgebauten Glycosidrest bedeutet.

Bevorzugte Beispiele für Verbindungen (1b) sind G- (CH₂CH₂O) -CH₂CH=CH₂ und G₂ -(CH₂CH₂O) -CH₂CH=CH₂, wobei G und G₂ die oben dafür angegebene Bedeutung haben.

In US 5,831,080 sind Glycosidreste aufweisende Organopolysiloxanverbindungen beschrieben.

Bevorzugt wird zur Herstellung der Verbindung (1b) ein Alkenylglucosid, besonders bevorzugt das (2-Allyloxy-ethoxy)-glucosid der Formel 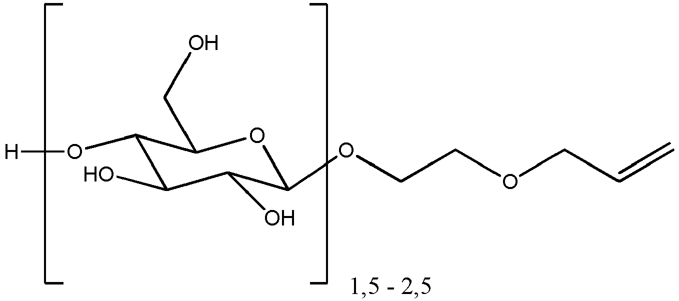 eingesetzt.

Dieses Alkenylglucosid wird nach dem in US 5,831,080 in Beispiel 1 (A) (Spalte 8, Zeilen 23 - 43) beschriebenem Verfahren hergestellt wird, mit dem Unterschied, dass das Lösemittel im letzten Schritt nicht vollständig abdestilliert wird, da dies zu einem bei Raumtemperatur glasartigen Material führen würde, sondern dass ein Lösemitteltausch auf 1,2-Propandiol durchgeführt wird. Das Produkt ist eine ca. 50%-ige Lösung des Alkenylglucosids in 1,2-Propandiol. Das Alkenylglucosid liegt teilweise aufkondensiert als Oligoglucosid vor.
Die Verbindungen (1b) werden daher vorzugsweise gelöst in einem organischen Lösungsmittel eingesetzt. Beispiele für organische Lösungsmittel sind Alkohole, wie Glycerin, 1,2-Propandiol, Methanol, Ethanol, n-Propanol und iso-Propanol; gesättigte Kohlenwasserstoffe, wie n-Pentan, n-Hexan, n-Heptan und n-Octan, und deren verzweigte Isomeren, wie Isododecan; Benzine, z. B. Alkangemische mit einem Siedebereich von 80°C bis 140°C bei 1020 hPa; ungesättigte Kohlenwasserstoffe wie 1-Hexen, 1-Hepten, 1-Octen und 1-Decen; aromatische Kohlenwasserstoffe wie Benzol, Toluol und Xylole; halogenierte Alkane mit 1 bis 6 Kohlenstoffatom(en), wie Methylenchlorid, Trichlorethylen und Perchlorethylen; Ether, wie Di-n-butyl-ether; Ester, wie Ethylacetat, Isopropylpalmitat und Isopropylmyristat (=Myristinsäure-isopropylester); Ketone, wie Methylethylketon und Cyclohexanon.

Bei den erfindungsgemäßen Organopolysiloxangelen werden ungesättigte Organopolysiloxanharze (1a) und Verbindungen (1b), die einen Glycosidrest und eine hydrosilylierbare Endgruppe aufweisen, in Mengen von vorzugsweise 4,5 bis 0,1 Mol, bevorzugt 2 bis 0,8 Mol, besonders bevorzugt 1,8 bis 1,1 Mol, Kohlenwasserstoffrest mit aliphatischer C-C-Mehrfachbindung je Mol Si-gebundenem Wasserstoff in Si-H funktionellen Organopolysiloxanen (2) und (2') eingesetzt, mit der Maßgabe, dass der gewichtsmäßige Anteil der Verbindung (1b) 0,1 - 10 Gew.-%, bevorzugt 0,1 - 5 Gew-%, besonders bevorzugt 0,3 - 2 Gew.-%, jeweils bezogen auf das Gesamtgewicht des Organopolysiloxangels ist.

Das gewichtsmäßige Verhältnis von MQ Harz zum Si-H-haltigen Organopolysiloxan in den in EP 1 132 430 A1 offenbarten Organopolysiloxangelen liegt im Bereich von 9 bis 7. Der hohe Anteil an vergleichsweise teurem Harz macht diese Gele vergleichsweise teuer. In den erfindungsgemäßen Organopolysiloxangelen liegt das gewichtsmäßige Verhältnis von ungesättigten Organopolysiloxanharzen (1) zu den Mischungen der Si-H-haltigen Organopolysiloxane (2) und (2') vorzugsweise im Bereich von 3 bis 0,1, bevorzugt im Bereich 2,5 bis 0,1, besonders bevorzugt im Bereich 2,0 bis 0,1.

Als Katalysator (3) können bei dem erfindungsgemäßen Verfahren die gleichen Katalysatoren eingesetzt werden, die auch bisher zur Förderung der Anlagerung von Si-gebundenem Wasserstoff an aliphatische Mehrfachbindung eingesetzt werden konnten. Bei den Katalysatoren handelt es sich vorzugsweise um ein Metall aus der Gruppe der Platinmetalle oder um eine Verbindung oder einen Komplex aus der Gruppe der Platinmetalle. Beispiele für solche Katalysatoren sind metallisches und feinverteiltes Platin, das sich auf Trägern wie Siliciumdioxid, Aluminiumoxid oder Aktivkohle befinden kann, Verbindungen oder Komplexe von Platin, wie Platinhalogenide, z. B. PtCl₄, H₂PtCl₆•6H₂O, Na₂PtCl₄•4H₂0, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus H₂PtCl₆•6H₂0 und Cyclohexanon, Platin-Vinyl-siloxankomplexe, wie Platin-1,3-Divinyl-1,1,3,3-tetramethyl-disiloxankomplexe mit oder ohne Gehalt an nachweisbarem anorganisch gebundenen Halogen, Bis-(y-picolin)-platindichlorid, Trimethylendipyridinplatindichlorid, Dicyclopentadienplatindichlorid, Dimethylsulfoxidethylenplatin-(II)-dichlorid, Cyclooctadien-Platindichlorid, Norbornadien-Platindichlorid, γ-picolin-Platindichlorid, Cyclopentadien-Platindichlorid, sowie Umsetzungsprodukte von Platintetrachlorid mit Olefin und primärem Amin oder sekundärem Amin oder primärem und sekundärem Amin, wie das Umsetzungsprodukt aus in 1-0cten gelöstem Platintetrachlorid mit sec.-Butylamin oder Ammonium-Platinkomplexe. Bevorzugte Hydrosilylierungskatalysatoren sind Platinverbindungen, die in einem zur Verwendung in kosmetischen Formulierungen geeigneten Lösungsmittel vorliegen.

Bevorzugt wird der Katalysator (3) in Mengen 1 bis 50 Gew.-ppm (Gewichtsteilen je Million Gewichtsteilen), besonders bevorzugt 2 bis 20 Gew.-ppm, jeweils berechnet als elementares Platin und bezogen auf das Gesamtgewicht der ungesättigten Organopolysiloxanharze (1), der Si-H funktionellen Organopolysiloxane (2) oder der Mischung der Si-H funktionellen Organopolysiloxane (2) und (2') und dem Verdünnungsmittel (4).

Die erfindungsgemäßen Organopolysiloxangele enthalten vorzugsweise 1 bis 98 Gew.-% Verdünnungsmittel, bevorzugt 50 bis 95 Gew.-% Verdünnungsmittel, bezogen auf das Gesamtgewicht der Organopolysiloxangele.

Nicht-reaktive oder relativ nicht-reaktive Verdünnungsmittel sind bevorzugt. Im Rahmen der vorliegenden Erfindung wird der Begriff "nicht-reaktiv" in Bezug auf die in Frage stehende Vernetzungsreaktion und die hierin eingesetzten Reaktanden verwendet. Ein relativ nichtreaktives Verdünnungsmittel ist weniger als ein Zehntel so reaktiv mit den Reaktanden der Vernetzungsreaktion im Vergleich zu den Reaktanden untereinander in der Vernetzungsreaktion.

Geeignete Beispiele von Verdünnungsmittel beinhalten cyclische und lineare Organopolysiloxane, organische Verdünnungsmittel oder Mischungen von Organopolysiloxanen und organischen Verdünnungsmitteln.

Das Organopolysiloxan kann ein einzelnes Organopolysiloxan oder eine Mischung von Organopolysiloxanen sein. Das Organopolysiloxan kann Alkyl-, Aryl-, Alkaryl- und Aralkylgruppen tragen. Solche Organopolysiloxane können beispielhaft durch Polydimethylsiloxan, Polydiethylsiloxan, Polymethylethylsiloxan, Polymethylphenylsiloxan und Polydiphenylsiloxan angegeben werden, sind aber nicht darauf beschränkt.

Möglich ist auch die Verwendung von funktionellen Organopolysiloxanen, beispielsweise acrylamidfunktionelle Siloxanfluide, acrylfunktionelle Siloxanfluide, amidfunktionelle Siloxanfluide, aminofunktionelle Siloxanfluide, carbinolfunktionelle Siloxanfluide, carboxyfunktionelle Siloxanfluide, chloralkylfunktionelle Siloxanfluide, epoxfunktionelle Siloxanfluide, glykolfunktionelle Siloxanfluide, ketalfunktionelle Siloxanfluide, mercaptofunktionelle Siloxanfluide, methylesterfunktionelle Siloxanfluide, perfluorofunktionelle Siloxanfluide und silanofunktionelle Siloxane.

Cyclische Polydimethylsiloxane können beispielhaft durch Hexamethylcyclotrisiloxan, Octamethylcyclotetrasiloxan, Decamethylcyclopentasiloxan und Dodecamethylcyclohexasiloxan angegeben werden, sind aber nicht darauf beschränkt.

Vorzugsweise ist das Organopolysiloxan ein Polydimethylsiloxan mit 2 bis 200 Si-Atomen, bevorzugt 2 bis 50 Si-Atomen, besonders bevorzugt sind lineare Polydimethylsiloxane mit einer Viskosität von 1,5 bis 50 mm²/s bei 25°C.

Als organische Verdünnungsmittel können aromatische Kohlenwasserstoffe, Alkohole, Aldehyde, Ketone, Amine, Ester, Ether, Alkylhalogenide oder aromatische Halogenide verwendet werden. Stellvertretende Beispiele sind Alkohole, wie Methanol, Ethanol, i-Propanol, Cyclohexanol, Benzylalkohol, 2-Octanol, Ethylenglykol, Propylenglykol und Glycerin; aliphatische Kohlenwasserstoffe, wie Pentan, Cyclohexan, Heptan, Lackbenzine; Alkylhalogenide wie Chloroform, Kohlenstofftetrachlorid, Perchlorethylen, Ethylchlorid und Chlorbenzol; aromatische Kohlenwasserstoffe, wie Benzol, Toluol, Ethylbenzol und Xylol; Ester von Carbonsäuren mit 2 bis 30 C-Atomen, wie Ethylacetat, Isopropylacetat, Ethylacetoacetat, Amylacetat, Isobutylisobutyrat, Benzylacetat, Isopropylpalmitat und Isopropylmyristat (=Myristinsäure-isopropylester); Ether, wie Ethylether, n-Butylether, Tetrahydrofuran und 1,4-Dioxan; Ketone, wie Aceton, Methylethylketon, Cyclohexanon, Diacetonalkohol, Methylamylketon und Diisobutylketon; Fettöle einschließlich mehrfach ungesättigter ω-3- und ω-6-Fettsäuren, und deren Ester; pflanzliche Öle, wie Erdnuss-, Oliven-, Palm-, Canola-, Maiskeim-, Soja-, Sonnenblumenöl und dergleichen; und natürliche und synthetische Öle oder öllösliche Feststoffe, wie verschiedene Mono-, Di- und Triglyceride, polyoxyalkylierte pflanzliche Öle, Lanolin, Lecithin und dergleichen; und Erdölkohlenwasserstoffe, wie Petrolatum, Mineralöl, Benzin, Petrolether. Diese Beispiele dienen der Erläuterung und sind nicht als Einschränkung zu verstehen.

Andere gemischte organische Verdünnungsmittel können auch verwendet werden, wie Acetonitril, Nitromethan, Dimethylformamid, Propylenoxid, Trioctylphosphat, Butyrolacton, Furfural, Pinienöl, Terpentin und m-Cresol.

Geeignete organische Verdünnungsmittel sind auch flüchtige Aromastoffe, wie Pfefferminzöl, Öl von grüner Minze, Menthol, Vanille, Zimtöl, Nelkenöl, Lorbeeröl, Anisöl, Eukalyptusöl, Thymianöl, Zedernöl, Öl von der Muskatnuss, Öl von Salbei, Kassiaöl, Kakao, Süßholzsaft, Stärkezuckersirup aus Mais mit hohem Fructosegehalt, Citrusöle, wie Zitrone, Orange, Limone und Grapefruit, Fruchtessenzen, wie Apfel, Birne, Pfirsich, Traube, Erdbeere, Himbeere, Kirsche, Pflaume, Ananas und Aprikose; und andere nützliche Aromastoffe einschließlich Aldehyde und Ester, wie Zimtsäureessigester, Zimtaldehyd, Eugenylformat, p-Methylanisol, Acetaldehyd, Benzaldehyd, Anisaldehyd, Citral, Neral, Decanal, Vanillin, Tolylaldehyd, 2,6-Dimethyloctanal und 2-Ethylbutyraldehyd.

Ein Teil oder das gesamte organische Verdünnungsmittel kann ein oder mehrere flüchtige Duftstoffe umfassen, wie natürliche Produkte und Parfumöle. Einige stellvertretende natürliche Produkte und Parfumöle sind Amber, Benzoin, Zibet, Nelke, Zedernöl, Jasmin, Mate, Mimose, Moschus, Myrrhe, Iris, Sandelholzöl und Vetiveröl; Aromachemikalien, wie Amylsalicylat, Amylzimtaldehyd, Benzylacetat, Citronellol, Cumarin, Geraniol, Isobornylacetat, Ambrette und Terpinylacetat, und verschiedene klassische Parfümölfamilien, wie die Blumenbouquetfamilie, die orientalische Familie, die Chyprefamilie, die Holzfamilie, die Citrusfamilie, die Canoefamilie, die Lederfamilie, die Gewürzfamilie und die Kräuterfamilie.

Das organische Verdünnungsmittel kann auch aliphatische oder alicyclische Kohlenwasserstoffe mit 4 bis 30 C-Atomen, vorzugsweise gesättigte Kohlenwasserstoffe, umfassen. Die aliphatischen Kohlenwasserstoffe können geradkettig oder verzweigt sein, und die alicyclischen Kohlenwasserstoffe können unsubstituierte cyclische Kohlenwasserstoffe oder aliphatische kohlenwasserstoffsubstituierte Kohlenwasserstoffe darstellen. Beispiele für geeignete Kohlenwasserstoffe sind n-Heptan, n-Octan, Isooctan, n-Decan, Isodecan, n-Dodecan, Isododecan, Cyclohexan, Cycloheptan, Cyclooctan, Methylcyclohexan, Dimethylcyclohexan, Ethylcyclohexan, Nonylcyclohexan und dergleichen. Auch diese Aufzahlung dient der Erläuterung und ist nicht als Einschränkung zu verstehen.

Weitere geeignete organische Verdünnungsmittel sind ölartige Polyether, wie Bis(alkyl)ether von niedermolekularen Glykolen, und flüssige oligomere und polymere Polyoxyalkylenglykole, deren Alkylmono- und -diether und Mono- und Dialkylester, wobei deren Verwendung aber nicht bevorzugt ist. Vorzugsweise wird der überwiegende Teil der Polyoxyalkylenglykole aus einem überwiegenden Teil (> 50 Mol-%) von Alkylenoxiden mit mehr als zwei Kohlenstoffatomen, d. h. Propylenoxid, 1,2- und 2,3-Butylenoxid, Tetrahydrofuran, Oxetan, Cyclohexenoxid und dergleichen, hergestellt.

Bevorzugte organische Verdünnungsmittel weisen eine Viskosität im Bereich von 0,5 bis 200 mm²/s (25°C) auf, wobei solche Verdünnungsmittel mit einem Siedepunkt im Bereich von 50°C bis 300°C besonders bevorzugt sind.

Es können zahlreiche Mischungen von Verdünnungsmittel verwenden werden, die lediglich auf diejenigen Zusammensetzungen beschränkt sind, bei denen nach der Herstellung des erfindungsgemäßen Organopolysiloxangels keine Phasentrennung auftritt.

Die Herstellung des Gels ist leicht durchführbar. Im Allgemeinen gibt man alle Bestandteile außer dem Katalysator zu, rührt langsam, bis sich das ungesättigte Organopolysiloxanharz gelöst hat, und setzt dann unter kontinuierlichem Rühren den Katalysator zu. Die Zusammensetzung kann bei Raumtemperatur belassen werden, bis sich ein Gel gebildet hat, oder erhitzt werden. Vorzugsweise erhitzt man die Zusammensetzung auf eine Temperatur zwischen 50°C und 130°C und bevorzugt zwischen 70°C und 120°C, bis die Mischung geliert oder fest wird. Die Gelierung erfolgt vorzugsweise innerhalb von zehn Stunden, bevorzugt innerhalb von drei Stunden. Es werden Organopolysiloxangele, welche für die Anwendung in kosmetischen Formulierungen geeignet sind, erhalten.
In einem besonders bevorzugten Verfahren, werden im ersten Teilschritt der Gelherstellung zunächst der/die Si-H-funktionelle(n) Vernetzer (2) und ggf. (2') und Verbindungen (1b), die einen Glycosidrest und eine hydrosilylierbare Endgruppe aufweisen, gemischt. Anschließend wird unter kontinuierlichem Rühren der Katalysator zugesetzt. Die Mischung wird vorzugsweise auf eine Temperatur zwischen 50°C und 130°C, bevorzugt zwischen 70°C und 120°C erhitzt, und 1 bis 480 Minuten, bevorzugt 1 bis 240, besonders bevorzugt 5 bis 60 Minuten bei dieser Temperatur gerührt. Danach wird im zweiten Teilschritt der Gelherstellung das ungesättigte Organopolysiloxanharz (1a) unter Rühren zugegeben und weiter gerührt, bis die Mischung geliert oder fest wird. Die Gelierung erfolgt vorzugsweise innerhalb von zehn Stunden, bevorzugt innerhalb von drei Stunden. Es werden Organopolysiloxangele, welche für die Anwendung in kosmetischen Formulierungen geeignet sind, erhalten.
In einem alternativen besonders bevorzugten Verfahren wird, sofern zwei Vernetzer mit unterschiedlichem Gehalt an Si-gebundenen Wasserstoff eingesetzt werden, zu Beginn, im ersten Teilschritt der Gelherstellung, nur einer der beiden Si-H-funktionellen Vernetzer und die Verbindung (1b), die einen Glycosidrest und eine hydrosilylierbare Endgruppe aufweisen, gemischt. Im zweiten Teilschritt der Gelherstellung wird vor, während oder nach der Zugabe des ungesättigten Organopolysiloxanharzes (1a) der zweite Si-H-funktionelle Vernetzer zugegeben.

In einem optionalen zweiten Verfahrensschritt wird das im ersten Verfahrensschritt erhaltene erfindungsgemäße Organopolysiloxangel unter Verwendung von standardmäßigen hochscherenden Mischtechniken bis zu einer cremigen Konsistenz homogenisiert. Dies kann durch intensives Mischen und Dispergieren in Rotor-Stator-Rührvorrichtungen, Kolloidmühlen, Hochdruckhomogenisatoren, Mikrokanälen, Membranen, Strahldüsen und ähnlichem, oder mittels Ultraschall erfolgen. Homogenisiergeräte und Verfahren sind z.B. in Ullmann's Encyclopedia of Industrial Chemistry, CD-ROM-Ausgabe 2003, Wiley-VCH Verlag, unter dem Stichwort "Emulsions" beschrieben.

In einem optionalen dritten Verfahrensschritt wird eine weitere Menge Verdünnungsmittel zu dem nach den ersten oder optionalen zweiten Verfahrensschritten erhaltenen Organopolysiloxangel gegeben. Dadurch ist es möglich, ausgehend von einem im ersten Verfahrensschritt erhaltenen "Basisgel" eine Vielzahl unterschiedlicher Gele herzustellen, die in ihrer Konsistenz und ihrem Eigenschaftsprofil in einem breiten Bereich variieren. Man kann dabei das gleiche Verdünnungsmittel verwenden, welches im ersten Verfahrensschritt verwendet worden ist, oder ein zweites Verdünnungsmittel beinhaltend die vorhergehend hierin als Verdünnungsmittel beschriebenen. Alternativ kann auch eine beliebige Mischung der vorhergehend hierin beschriebenen Verdünnungsmittel und/oder ein aktiver Wirkstoff für die Körperpflege oder Gesundheitspflege oder eine Mischung eines aktiven Wirkstoffs für die Körperpflege oder Gesundheitspflege mit einem oder mehreren der hierin beschriebenen Verdünnungsmitteln zugegeben werden, mit der Maßgabe, dass keine Phasentrennung auftritt.

Ein "aktiver Wirkstoff für die Körperpflege oder Gesundheitspflege" bedeutet im vorliegenden Zusammenhang eine beliebige Verbindung oder Mischung von Verbindungen, die im Fachgebiet als Zusatzstoffe in Körperpflegeformulierungen bekannt sind und die typischerweise zugesetzt werden, um das Haar oder die Haut zu behandeln, um einen kosmetischen und/oder ästhetischen Nutzen zu erzielen, eine beliebige Verbindung oder Mischung von Verbindungen, die im Fachgebiet bekannt sind, um einen pharmazeutischen oder medizinischen Nutzen zu erzielen; eine beliebige Verbindung, mit der eine pharmakologische Wirksamkeit oder einen sonstigen Effekt bei der Diagnose, Heilung, Linderung, Behandlung oder Vorbeugung von Krankheiten erzielt werden soll, oder um die Struktur oder eine beliebige Funktion des Körpers eines Menschen oder eines Tiers zu beeinflussen; und jede beliebige Verbindung, die bei der Herstellung von Arzneimittelprodukten eine chemische Veränderung erfahren kann und die in Arzneimitteln in modifizierter Form vorliegen kann, um die angegebene Wirksamkeit oder den angegebenen Effekt hervorzurufen. So umfasst ein "aktiver Wirkstoff für die Körperpflege oder Gesundheitspflege" einen aktiven Wirkstoff oder aktiven Arzneimittelbestandteil wie allgemein von der United States Department of Health & Human Services Food and Drug Administration, Titel 21, Kapitel I, des Code of Federal Regulations, Teile 200 - 299 sowie Teile 300-499 definiert, ist jedoch nicht hierauf beschränkt.

Die aktiven Wirkstoffe für die Körperpflege oder Gesundheitspflege sind vorzugsweise ausgewählt aus der Gruppe der fett- oder öllöslichen Vitamine, öllöslichen Arzneimittel, wobei Antiaknemittel, antibakterielle Mittel, fungizide Mittel, entzündungshemmende Mittel, schuppenflechtebekämpfende Mittel, Betäubungsmittel, juckreizlindernde Mittel, hautentzündungshemmende Mittel und Mittel, die im Allgemeinen als Sperrfilme betrachtet werden besonders bevorzugt sind, und öllöslichen UV-Absorber.

Nützliche aktive Bestandteile zur Verwendung im dritten Verfahrensschritt gemäß der Erfindung umfassen fett- als auch öllösliche Vitamine. Nützliche, öllösliche Vitamine umfassen, sind, aber nicht beschränkt auf, Vitamin A₁, RETINOL, C₂- bis C₁₈-Ester von RETINOL, Vitamin E, TOCOPHEROL, Ester von Vitamin E und Mischungen derselben. RETINOL umfasst trans-RETINOL, 13-cis-RETINOL, 11-cis-RETINOL, 9-cis-RETINOL und 3,4-Didehydro-RETINOl. Das öllösliche Vitamin kann in der Zusammensetzung gemäß der Erfindung in Mengen von 0,01 bis 50 Gewichtsprozent verwendet werden.

Es sollte bemerkt werden, dass RETINOL ein International Nomenclature Cosmetic Ingredient Name (INCI), vergeben von The Cosmetic, Toiletry and Fragrance Association (CTFA), Washington DC, für Vitamin A ist. Andere geeignete Vitamine und die INCI-Namen für die in Betracht stehenden Vitamine, die hierin umfasst sind, sind RETINYL ACETAT, RETINYL PALMITATE, RETINYL PROPIONATE, a- TOCOPHEROL,TOCOPHERSOLAN,TOCOPHERYL ACETATE, TOCOPHERYLLINOLEATE, TOCOPHERYLNICOTINATE und TOCOPHERYL SUCCINATE.

Einige Beispiele für kommerziell erhältliche Produkte, die zur Verwendung hierin geeignet sind, sind Vitamin-A-Acetat, Fluka Chemie AG, Buchs, Schweiz; CIOVI-OX T-50, ein Vitamin E-Produkt von Henkel Corporation, La Grange, Illinois; COVI-OX T-70, ein anderes Vitamin-E-Produkt von Henkel Corporation, La Grange Illinois, und Vitamin-E-Acetat, ein Produkt von Roche Vitamins & Fine Chemicals, Nutley, New Jersey.

Stellvertretende Beispiele für einige geeignete öllösliche Arzneimittel, welche als aktive Bestandteile im dritten Verfahrensschritt gemäß der Erfindung zugefügt werden können, sind Clonidin, Scopolamin, Propranolol, Estradiol, Phenylpropanolaminhydrochlorid, Ouabain, Atropin, Haloperidol, Isosorbid, Nitroglycerin, Ibuprofen, Ubichinone, Indomethacin, Prostaglandine, Naproxen, Salbutamol, Guanabenz, Labetalol, Pheniramin, Metrifonat und Steroide.

Ebenso hierin umfasst als ein Arzneimittel für die Zwecke der vorliegenden Erfindung sind Antiaknemittel wie Benzoylperoxid, Triclosan und Tretinoin; antibakterielle Mittel wie Chlorhexidingluconat; fungizide Mittel wie Miconazolnitrat; entzündungshemmende Mittel wie Salicylsäure; corticosteroide Arzneimittel; nichtsteroide entzündungshemmende Mittel wie Diclofenac; schuppenflechtebekämpfende Mittel wie Clobetasolpropionat und Retinoide, Betäubungsmittel wie Lidocain; juckreizlindernde Mittel wie Polidocanol; hautentzündungshemmende Mittel wie Prednisolon und Mittel, die im Allgemeinen als Sperrfilme betrachtet werden.

Stellvertretende Beispiele für öllösliche UV-Absorber, welche als aktive Bestandteile im dritten Verfahrensschritt gemäß der Erfindung zugefügt werden können, sind 1-(4-Methoxyphenyl)-3-(4-tert-butylphenyl)propan-1,3-dione (INCI: Butyl Methoxydibenzoylmethan), 2-Ethylhexyl-(2E)-3-(4-methoxyphenyl)prop-2-enoat (INCI: Octyl Methoxycinnamat), 4-Hydroxy-2-methoxy-5-(oxo-phenylmethyl)benzenesulfonic acid (INCI: Benzophenon-4), 2-Hydroxy-4-methoxybenzophenon-5-sulfonsäure Natriumsalz (INCI: Benzophenon-5) und 2-Ethylhexyl-2-hydroxybenzoat(INCI: Ethylhexylsalicylat).

Bevorzugt wird in einem vierten Verfahrensschritt das nach dem ersten oder optionalen zweiten oder optionalen dritten Verfahrensschritt erhaltene erfindungsgemäße Organopolysiloxangel unter Verwendung von standardmäßigen hochscherenden Mischtechniken bis zu einer cremigen Konsistenz homogenisiert. Hierfür geeignete Technologien sind oben genannt. Wenn im optionalen dritten Verfahrensschritt eine zusätzliche Menge Verdünnungsmittel zugegeben worden ist, so wird dieses im vierten Verfahrensschritt homogen im Gel verteilt. Das Gel quillt und verändert seine Weichheit.

Unter "cremig" in Bezug auf das Gel ist zu verstehen, dass das Ausgangsgel bis zu einer cremigen Konsistenz geschert worden ist. Das resultierende cremige Gel kann je nachdem gießbar oder verhältnismäßig steif sein. Durch das Attribut "cremig" unterscheiden sich diese gescherten Gele, die transparent oder opak sein können, von den unmittelbar durch Gelierung der reaktiven Bestandteile hergestellten Gelen.

Unter "lagerstabil" im Rahmen dieser Erfindung ist zu verstehen, dass sich die gebildeten Organopolysiloxangele innerhalb von 6 Monaten Lagerung bei Raumtemperatur nicht in zwei oder mehr Phasen entmischen und sich die Weichheit des Gels in diesem Zeitraum nicht wesentlich ändert.

Beim Einmischen von polaren oder hydrophilen Lösungsmitteln, wie Wasser oder Glycerin, in die erfindungsgemäßen, hydrophil modifizierten Gele wird das Lösemittel zunächst unter Ausbildung einer einphasigen, homogenen Mischung ins Gel aufgenommen und es werden bei Verwendung von Glycerin feste Cremes bzw. bei Verwendung von Wasser gallertartige Massen erhalten. Wird eine bestimmte Menge des polaren oder hydrophilen Lösungsmittels überschritten, so wird ein ZweiPhasen-Gemisch aus einer weißen Creme und einer klaren Flüssigkeit erhalten, d.h. ab einer bestimmten Menge des polaren oder hydrophilen Lösungsmittels ist das Gel "gesättigt" und nimmt keine weitere Menge des entsprechenden polaren oder hydrophilen Lösungsmittels mehr auf.
Hierin unterscheiden sich die erfindungsgemäßen Gele von Emulsionen, die verdünnungsstabil sind, d.h. es können beliebige Mengen Verdünnungsmittel zugegeben werden und es entsteht eine niederviskose "Milch". Die erfindungsgemäßen Gele unterscheiden sich daher auch von sogenannten "selbstemulgierenden Elastomergelen".

Ein wesentlicher Vorteil der erfindungsgemäßen Organopolysiloxangele ist daher ihre verbesserte Kompatibilität mit polaren oder hydrophilen organischen Substanzen, beispielsweise Glycerin, und sogar Wasser. Diese wichtigen Kosmetikinhaltsstoffe sind mit herkömmlichen Organopolysiloxangelen nicht, oder nur in sehr geringen Mengen mischbar. Die erfindungsgemäßen Organopolysiloxangele sind in der Lage, äußerst polare Stoffe, wie Wasser oder Glycerin, unter Beibehalt der viskosen Gelstruktur und Ausbildung einer einphasigen, homogenen Mischung aufzunehmen.
Durch ihre verbesserte Kompatibilität mit polaren und hydrophilen organischen Substanzen und sogar Wasser, zeigen die erfindungsgemäßen Organopolysiloxangele auch in wasser- oder alkoholbasierten Formulierungen einen verdickenden Effekt und verleihen solchen Formulierungen ein bevorzugtes seidiges Hautgefühl.

Gegenstand der Erfindung sind daher einphasige, homogene Mischungen enthaltend
(a) erfindungsgemäße Glycosidreste aufweisende Organopolysiloxangele und
(b) polare oder hydrophile Lösungsmittel.

Beispiele für polare oder hydrophile Lösungsmittel sind Wasser, Glycerin, Ethylenglycol, Diethylenglycol, Propylenglycol und deren Gemische, wobei Wasser und Glycerin besonders bevorzugt sind.

Die erfindungsgemäßen Organopolysiloxangele können Wasser in Mengen von vorzugsweise mindestens 5 Gew.-%, bevorzugt mindestens 10 Gew.-%, und vorzugsweise höchstens 400 Gew.-%, bevorzugt höchstens 200 Gew.-%, jeweils bezogen auf das Gesamtgewicht der Organopolysiloxangele, aufnehmen.
Glycole können sie in Mengen von vorzugsweise mindestens 20 Gew.-%, bevorzugt mindestens 25 Gew.-%, und vorzugsweise höchstens 600 Gew.-%, bevorzugt höchstens 300 Gew.-%, jeweils bezogen auf das Gesamtgewicht der Organopolysiloxangele aufnehmen.

Dem Fachmann ist verständlich, dass das Aufnahmevermögen für Verdünnungsmittel aufgrund der dreidimensionalen Netzwerkstruktur von Organopolysiloxangelen im Allgemeinen beschränkt ist und in Abhängigkeit von der Netzwerkstruktur und -zusammensetzung variieren kann. Ist das Aufnahmevermögen für Verdünnungsmittel überschritten, so ist die Bildung einer Verdünnungsmittelphase neben einer Gelphase zu erkennen. Bei den erfindungsgemäßen Organopolysiloxangelen zeigt sich die allgemeine Tendenz, dass das Aufnahmevermögen von hydrophilen Substanzen bei vergleichbaren Gelformulierungen höher ist, wenn der Substitutionsgrad mit Verbindungen (1b), die einen Glycosidrest und eine hydrosilylierbare Endgruppe aufweisen, höher ist. Dies öffnet die Möglichkeit, die Hydrophilie des erfindungsgemäßen Organopolysiloxangels gezielt nach dem Anforderungsprofil der Anwendung anzupassen und zu optimieren.

Dem erfindungsgemäßen Organopolysiloxangel wird vorzugsweise ein Hydrosilylierungskatalysatorgift oder ein SiH-Quencher zugesetzt, wodurch die Nachhärtung, die durch verbleibende vernetzende Hydrosilylierungsreaktionen, die in den Siliconelastomeren auftreten, bewirkt wird, beendet wird. Beispielhaft für Hydrosilylierungskatalysatorgifte oder SiH-Quencher, welche geeignet sind um die Nachhärtung zu beenden, sind schwefelorganische Verbindungen. Weitere geeignete Verbindungen sind in US 6,200,581 genannt. Bevorzugte Hydrosilylierungskatalysatorgifte sind Mercaptoalkylorganopolysiloxanen, besonders bevorzugt sind mercaptopropylfunktionelle Silsesquisiloxane oder mercaptopropylfunktionelle Polyorganosiloxane, welche vorzugsweise in Mengen von 200 bis 1,0 Mol, bevorzugt 50 bis 1,5 Mol, besonders bevorzugt 20 bis 2,0 Mol, Mercaptogruppen je Mol Platinatome eingesetzt werden. Die Zugabe des Hydrosilylierungskatalysatorgifts oder des SiH-Quenchers kann beliebig in einem oder mehreren der genannten Verfahrensschritte erfolgen.

Die erfindungsgemäßen Organopolysiloxangele sind besonders bevorzugt geeignet für kosmetische Anwendungen, und werden daher bevorzugt in kosmetischen Zusammensetzungen eingesetzt. Sie eignen sich aber auch für andere Anwendungen, beispielsweise für medizinische und technische Anwendungen.

Die Organopolysiloxangele haben besonderen Wert in Körperpflegeprodukten. Sie können sanft auf der Haut verteilt werden und können daher alleine verwendet werden oder mit anderen Körperpflegeproduktbestandteilen gemischt werden, um eine Vielzahl von Körperpflegeprodukten zu bilden.

Beispiele von Körperpflegeproduktbestandteilen sind Ester, Wachse, Öle und Fette von tierischem oder pflanzlichem Ursprung, Fettalkohole, Fettsäuren, Alkylester von Fettsäuren, Kohlenwasserstoffe und -wachse, Wasser, organische Lösungsmittel, Parfüme, oberflächenaktive Mittel, öllösliche Vitamine, wasserlösliche Vitamine, öllösliche Arzneimittel, wasserlösliche Arzneimittel, UV-Absorber, aktive pharmazeutische Verbindungen und andere.

Insbesondere sind die erfindungsgemäßen Organopolysiloxangele in Antiperspirantien und Deoperspirantien geeignet, da sie ein trockenes Gefühl zurücklassen und die Haut nicht während der Verdampfung abkühlen. Sie sind gleitfähig und verbessern die Eigenschaften von Hautcremes, Hautpflegelotionen, Moisturizern, Gesichtsbehandlungen wie z.B. Akne- oder Faltenentfernern, Körper- und Gesichtsreinigern, Badeölen, Parfüms, Kölnisch Wasser, Sachets, Sonnenschutzmittel, Preshave- und Aftershave-Lotionen, flüssigen Seifen, Rasierseifen und Rasierschäumen. Sie können in Haarshampoos, Haarkonditionierern, Haarsprays, Mousses, Dauerwellenmittel, Haarentfernungsmittel und Nagelhautabdeckungen verwendet werden, um Glanz und Trockengleiten zu verbessern und Konditioniervorteile bereitzustellen.

In Kosmetika fungieren Sie als Verteilungsmittel für Pigmente in Make-ups, Farbkosmetika, Grundierungen, Rouge, Lippenstiften, Lippenbalsam, Lidstrich, Mascara, Ölentfernern und Farbkosmetikentfernern. Sie sind als Verabreichungssysteme für hierin beispielhaft genannte öllösliche aktive Bestandteile wie z.B. Vitamine, Arzneimittel und UV Absorber geeignet. Wenn sie in Stiften, Gelen, Lotionen, Cremes, Roll-ons eingesetzt werden, verleihen die Elastomere ein trockenes, seidig sanftes Gefühl. Wenn in Kosmetika und anderen Hautpflegeprodukten eingebracht, verleihen die Elastomere einen Mattierungseffekt.

Zusätzlich zeigen die Organopolysiloxangele eine Vielzahl vorteilhafter Eigenschaften wie z.B. Klarheit, Lagerstabilität und Einfachheit der Herstellung. Daher haben sie einen breiten Anwendungsbereich, insbesondere in Antiperspirantien, Deodorantien, Hautpflegeprodukten, in Parfüms als Träger und für die Haarkonditionierung, beispielsweise in Hair-Balm oder Hair-Mask Conditioner.

Die Organopolysiloxangele haben Verwendung über den Körperpflegebereich hinaus, einschließlich deren Verwendung als Füllstoff oder Isolierungsmaterial für elektrische Kabel, Boden- oder Wasserbarrieren für Bodenstabilisierung oder als Ersatz für Epoxymaterialen die in Bauteilen in der elektronischen Industrie verwendet werden. Sie sind ebenfalls als Träger für vernetzte Siliconkautschukteilchen geeignet. In diesen Anwendungen erlauben sie (i) die Einfachheit des Einbringens von Teilchen in solche Silicon- oder organische Phasen, wie Dichtmittel, Farben, Beschichtungen, Fetten, Klebstoffen, Antischaummitteln und Gießharzverbindungen, und (ii) stellen modifizierte rheologische, physikalische oder energieabsorbierende Eigenschaften solcher Phasen, entweder in ihrem reinen oder in ihrem Endzustand, bereit.

Zusätzlich sind die Organopolysiloxangele in der Lage, als Träger für Pharmazeutika, Biozide, Herbizide, Pestizide und andere biologische aktive Substanzen zu wirken.

Weiterhin finden die Zusammensetzungen Anwendung als Additive für nichtgewebte Trägersubstrate auf Cellulosebasis oder nichtgewebte synthetische Trägersubstrate, die in feuchten Reinigungstüchern wie Feuchttüchern, feuchten Papiertüchern und feuchten Handtüchern verwendet werden, die im Allgemeinen für Körperhygiene-·und Haushaltsreinigungszwecke vermarktet werden.

Die erfindungsgemäßen Organopolysiloxangele können als Träger zur gesteuerten und leicht regulierten Freisetzung einer flüchtigen aktiven organischen Substanz in die freie Atmosphäre verwendet werden, wenn sie mit dieser vermischt werden. Die flüchtige Substanz kann insbesondere ein Parfüm sein oder ein Insektizid oder ein Stoff, der Insekten abwehrt.
In dieser Verwendung finden die erfindungsgemäßen Organopolysiloxangele breite Anwendung, beispielsweise bei der Ausrüstung von Fasern, Textilien und Stoffen aus Baumwolle oder Kunstfasern, Geweben, Tüchern, auch Papiertüchern, Toilettenpapier oder Wischpapier, wie Servietten oder Küchenrolle, oder Vlies, zur langanhaltenden, kontrollierten Beduftung oder Insektenabwehr. Die Mischung aus dem erfindungsgemäßen Organopolysiloxangele und der flüchtigen aktiven organischen Substanz kann auch in Waschmaschinen und Wäschetrockner direkt als solche oder als Zusatz zu Waschmitteln und Weichspülern auf Stoffe und Textilien appliziert werden.

Die Verwendung der erfindungsgemäßen Organopolysiloxangele als Träger zur gesteuerten und leicht regulierten Freisetzung einer flüchtigen aktiven organischen Substanz findet insbesondere Anwendung in den oben genannten kosmetischen Anwendungen, wo sie einen Zusatzeffekt zum dort beschrieben Effekt bewirken können, indem sie beispielsweise ein Parfüm gesteuert abgeben. Die erfindungsgemäßen Organopolysiloxangele können auch in Insektenabwehrpräparaten eingesetzt werden, wo sie ein Insektizid oder ein Stoff, der Insekten abwehrt, abgeben. Solche Produkte können beispielsweise direkt auf die Haut oder auf die Kleidung aufgebracht werden.

In einer weiteren Anwendung können die Mischung aus dem erfindungsgemäßen Organopolysiloxangele und der flüchtigen aktiven organischen Substanz zur kontrollierten Beduftung oder Insektenabwehr in geschlossenen Räumen, wie zum Beispiel in Wohnräumen, Geschäftsräumen, WCs oder Fahrzeugen wie Busse und Autos, eingesetzt werden.

### Gelzubereitung, Allgemeine Vorschrift (A und B)

Nach Methode A wird zunächst ein "Basisgel" hergestellt, welches nach der Gelierung durch Zugabe einer weiteren Menge Verdünnungsmittel verdünnt wird. Methode B unterscheidet sich von Methode A grundsätzlich dadurch, dass von Beginn an die volle Menge Verdünnungsmittel zugegeben wird. Es findet keine nachträgliche Verdünnung des erhaltenen Gels statt.
Die Hydrosilylierung wird in einem Schritt H1 oder in zwei aufeinanderfolgenden Schritten H1 und H2 durchgeführt.

### Vorschrift A:

Ein 2000 ml-Reaktionsgefäß aus Glas wird mit einem Kühler mit aufgesetzter Stickstoffeinleitung, Heizmantel, Ankerrührer und Temperaturregelung ausgestattet. Das Reaktionsgefäß wird vor Beginn der Reaktion 5 min mit Stickstoff gespült. Eine entsprechende Menge Verdünnungsmittel, der(die) Si-H-haltige(n) Vernetzer für Verfahrensschritt H1 und (2-Allyloxy-ethoxy)-glucosid, als 50%-ige Lösung in 1,2-Propandiol, werden zugegeben. Anschließend werden 5 ppm Hydrosilylierungskatalysator zugegeben und die Reaktionsmischung wird bei einer Rührgeschwindigkeit von etwa 200 U/min 1 h auf 95°C erhitzt. Nun wird zunächst der optionale Si-H-haltige(n) Vernetzer für Verfahrensschritt H2 und anschließend das ungesättigte Organopolysiloxanharz zugegeben und bis zur vollständigen Lösung des Harzes gerührt. 5 ppm Hydrosilylierungskatalysator werden zugegeben und die Reaktionsmischung wird bei einer Rührgeschwindigkeit von etwa 200 U/min 2,5 Stunden bei 95 °C gerührt. Anschließend wird der Heizmantel entfernt und unter reduzierter Rührgeschwindigkeit (ca. 50 U/min) auf Raumtemperatur abgekühlt und Katalysatorgift zugesetzt. Das erhaltene Gel wird unter Schwenken eine Minute mit einem ULTRA-TURRAX® T 50 bei 6000 U/min homogenisiert. Man erhält ein "Basisgel", welches eine cremige bis feste oder bröckelige Konsistenz haben kann und für die Verwendung in Kosmetikprodukten geeignet ist.

Für die Verdünnung wird die gewünschte Menge Verdünnungsmittel zugegeben und bei 50 U/min mit dem Ankerrührer gerührt, bis das Verdünnungsmittel vollständig vom Gel aufgenommen worden ist (ca. 10 Minuten). Anschließend wird wie erneut unter Schwenken eine Minute mit einem ULTRA-TURRAX® T 50 bei 6000 U/min homogenisiert. Auf diesem Weg erhält man ein lagerstabiles, cremiges, transparentes, transluzentes oder opakes Gel mit sehr glatter Konsistenz, das zur Verwendung in Kosmetikprodukten geeignet ist.

### Vorschrift B:

Ein 2000 ml-Reaktionsgefäß aus Glas wird mit einem Kühler mit aufgesetzter Stickstoffeinleitung, Heizmantel, Ankerrührer und Temperaturregelung ausgestattet. Das Reaktionsgefäß wird vor Beginn der Reaktion 5 min mit Stickstoff gespült. Das Verdünnungsmittel, der(die) Si-H-haltige(n) Vernetzer für Verfahrensschritt H1 und (2-Allyloxy-ethoxy)-glucosid, als 50%-ige Lösung in 1,2-Propandiol, werden zugegeben. Anschließend wird 5 ppm Hydrosilylierungskatalysator zugegeben und die Reaktionsmischung wird bei einer Rührgeschwindigkeit von etwa 200 U/min 1 h auf 95 °C erhitzt. Nun wird zunächst der optionale Si-H-haltige(n) Vernetzer für Verfahrensschritt H2 und anschließend das ungesättigte Organopolysiloxanharz zugegeben und bis zur vollständigen Lösung des Harzes gerührt. 5 ppm Hydrosilylierungskatalysator werden zugegeben und die Reaktionsmischung wird bei einer Rührgeschwindigkeit von etwa 200 U/min 2,5 Stunden bei 95 °C gerührt. Anschließend wird der Heizmantel entfernt und unter reduzierter Rührgeschwindigkeit (ca. 50 U/min) auf Raumtemperatur abgekühlt und Katalysatorgift zugesetzt. Das erhaltene Gel wird unter Schwenken zwei Minuten mit einem ULTRA-TURRAX® T 50 bei 6000 U/min homogenisiert. Auf diesem Weg erhält man ein lagerstabiles, cremiges, transparentes, transluzentes oder opakes Gel mit sehr glatter Konsistenz, das zur Verwendung in Kosmetikprodukten geeignet ist.

(2-Allyloxy-ethoxy)-glucosid wird nach dem in US 5,831,080 in Beispiel 1 (A) (Spalte 8, Zeilen 23 - 43) beschriebenen Verfahren hergestellt.

### Analytische Methoden:

Die Viskosität der Organopolysiloxangele wurden nach DIN EN ISO 3219 bei Schergeschwindigkeit 1/s und 25°C bestimmt.

Die Viskosität der Organopolysiloxane, wie Si-H-haltigen Vernetzer, Organopolysiloxanharze und Polydimethylsiloxane, wurden nach DIN 53019 im linearen Bereich bei 25°C bestimmt.

Die Jodzahl wurde nach DIN 53241-1 gemäß dem Verfahren nach Wijs bestimmt.

Gelpermeationschromatografie zur Bestimmung des gewichtsmittleren Molekulargewichts Mw wurde durchgeführt nach ISO 16014-1 und ISO 16014-3.

### Beispiel 1 - 14 und Vergleichsbeispiel V1 - V6:

Nach den Vorschriften A und B wurde eine Reihe von Gelen hergestellt. Die Eigenschaften der in den Beispielen und Vergleichsbeispielen verwendeten Si-H-funktionellen Vernetzer sind in Tabelle 1 dargestellt. Vernetzer 1 und 2 (Tabelle 1) wurden im Schritt H1 zugegeben, Vernetzer 3 (Tabelle 1) wurde im Schritt H2 zugegeben. Die verwendeten Substanzen, deren Mengen und die Eigenschaften der hergestellten Gele sind in den nachstehenden Tabellen 2 bis 5 wiedergegeben.

**TABELLE 1 - EIGENSCHAFTEN DER IN BEISPIEL 1 - 14 und Vergleichsbeispielen V1 - V6 VERWENDETEN SI-H-HALTIGEN VERNETZER:**

| **Nr.** | **Verteilung a: (b+c)** | **Summe a+b** | **Viskosität (mm²/s bei 25°C)** | **% H** |
|---|---|---|---|---|
| | | | | |
| 1 | 2 : 1 | 138 | 331 | 0.47 |
| 2 | 9 : 1 | 60 | 58 | 0.14 |
| 3 | 55 : 1 | 134 | 321 | 0.026 |

**Beispiele 1 - 7** sind Beispiele für erfindungsgemäße Gele, bei denen ein Vernetzer mit einem sehr niedrigen Gehalt an Si-H Gruppen verwendet wird, als einziger Vernetzer oder in Kombination mit einem weiteren Vernetzer. Als Verdünnungsmittel wurde flüchtiges, lineares Polydimethylsiloxan (2 mm²/s bei 25°C) gewählt. Es werden lagerstabile, cremige Gele erhalten, welche für die Verwendung in kosmetischen Formulierungen geeignet sind und signifikante Mengen hydrophiler Flüssigkeit aufnehmen können. Die Vergleichsbeispiele V1, V2 und V3 zeigen Gele im gleichen Verdünnungsmittel, wie es in den Beispielen 1 - 7 verwendet wurde. Vergleichsbeispiel V1 enthält jedoch ausschließlich einen Vernetzer mit sehr hohem Gehalt an Si-H-Gruppen, wie in EP 1 132 430 A1 offenbart. Man erhält ein niederviskoses, fließendes Gel mit öligem Gefühl, welches für die beschriebenen Anwendungen nicht geeignet ist. Vergleichsbeispiel V2 enthält einen Vernetzer mit sehr hohem Gehalt an Si-gebundenen Wasserstoff in Kombination mit 20 Gew.-% eines weniger funktionellen Vernetzers. Man erhält kein Gel, sondern eine Flüssigkeit. Vergleichsbeispiel V3 hat eine zum Beispiel 3 ähnliche Rezeptur, wurde jedoch ohne die erfindungsgemäße Verbindung (1b), also ohne eine Verbindung, die einen Glycosidrest und eine hydrosilylierbare Endgruppe aufweist, hergestellt. Das Gel ist cremig, lagerstabil und transparent, nimmt jedoch weder Wasser noch Glycerin auf, wie die Vergleichsbeispiele V7 (Tabelle 7) und V13 (Tabelle 8) zeigen.

**TABELLE 2 - ELASTOMERGEL FORMULIERUNGEN:**

| Beispiel: | | V1 | V2 | 1 | 2 | 3 | 4 | V3 |
|---|---|---|---|---|---|---|---|---|
| Verdünnungsmittel (g) | Polydimethyl -siloxan (2 mm²/s)¹ | 760 | 770 | 760 | 760 | 760 | 770 | 810 |
| ungesättigtes Silikonharz² (g) | | 206,6 | 506 | 157,4 | 118,4 | 70,4 | 118 | 90,3 |
| Si-H-haltiger Vernetzer (g) | Nr. 1 (0,46 % H) | 23 | 21,2 | | | | | |
| | Nr. 2 (0,14 % H) | | 5,3 | 58,9 | 32,4 | | 32,4 | |
| | Nr. 3 (0,026 % H) | | | 14,3 | 78,6 | 159,2 | 78,6 | 169 |
| (2-Allyloxy-ethoxy) -glucosid (g) | | 5 | 5 | 5 | 5 | 5 | 8 | - |
| 1,2-Propandiol (g) | | 5 | 5 | 5 | 5 | 5 | 8 | - |
| Platingift (g) | Mercaptoöl³ | 6,9 | 6,9 | 6,9 | 6,9 | 6,9 | 6,9 | 3,8 |
| Katalysator (Gew.-ppm) | Platin-Komplex⁴ | 5 + 5 | 5 + 5 | 5 + 5 | 5 + 5 | 5 + 5 | 5 + 5 | 5 |
| Ansatzgröße (g) | | 1006,5 | 1019,4 | 1007,5 | 1006,3 | 1006,5 | 1021,9 | 1073,1 |
| Mol Vinyl / Mol Si-H | | 1,4 | 1,41 | 1,43 | 1,49 | 1,61 | 1,59 | 1,58 |
| Viskosität (mPa*s bei 25°C) | | 18800 | 1260 | 185000 | 236000 | 446000 | 124000 | 473000 |
| Eigenschaften | | zähflüssig | flüssig | cremig, standfest | cremig, standfest | cremig, standfest | cremig, standfest | cremig, standfest |
| Aussehen | | transluzent | transluzent | transluzent | transluzent | transluzent | transparent | transparent |
| Lagerstabil | | ja | ja | ja | ja | ja | ja | ja |
| Gew.-% Elastomer im "Basisgel" (bei Methode A) | | - | - | - | - | - | - | - |
| Gew.-% Elastomer im fertigen Gel | | 24 | 24 | 24 | 24 | 24 | 24 | 24 |
| Verwendete Vorschrift | | B | B | B | B | B | B | B |

| | | | | | | | | |
|---|---|---|---|---|---|---|---|---|
| ¹PSF-2cSt Pure Silicone Fluid Dodecamethylpentasiloxane zu beziehen bei CLEARCO PRODUCTS CO. INC., U.S.A.; Viskosität bei 25°C; ²Verhältnis M / M^{Vi} / Q = 7,6 / 1 / 11,4, Mₙ = 2570, M_{w} = 5440, Jodzahl = 18; ³Polysiloxan mit 3-Mercaptopropylgruppen; Viskosität 190 mm²/s bei 25 °C, Mercaptangehalt 0,29 Gew.-%; ⁴WACKER® CATALYST OL zu beziehen bei Wacker Chemie AG; | | | | | | | | |

**TABELLE 3 - ELASTOMERGEL FORMULIERUNGEN:**

| Beispiel: | | 5 | 6 | 7 |
|---|---|---|---|---|
| Verdünnungsmittel (g) | Polydimethylsiloxan (2 mm²/s)¹ | 950 | 1250 | 1470 |
| ungesättigtes Silikonharz² (g) | | 157,4 | 118,4 | 70,4 |
| Si-H-haltiger Vernetzer (g) | Nr. 1 (0,46 % H) | | | |
| | Nr. 2 (0,14 % H) | 58,9 | 32,4 | |
| | Nr. 3 (0,026 % H) | 14,3 | 78,6 | 159,2 |
| (2-Allyloxy-ethoxy) -glucosid (g) | | 5 | 5 | 5 |
| 1,2-Propandiol (g) | | 5 | 5 | 5 |
| Platingift (g) | Mercaptoöl³ | 6,9 | 6,9 | 6,9 |
| Katalysator (Gew.-ppm) | Platin-Komplex⁴ | 5 + 5 | 5 + 5 | 5 + 5 |
| Ansatzgröße (g) | | 1197,5 | 1496,3 | 1006,5 |
| Mol Vinyl / Mol Si-H | | 1,43 | 1,49 | 1,61 |
| Viskosität (mPa*s bei 25°C) | | 86100 | 108000 | 79000 |
| Eigenschaften | | cremig, standfest | cremig, standfest | cremig, standfest |
| Aussehen | | transluzent | transluzent | transluzent |
| Lagerstabil | | ja | ja | ja |
| Gew.-% Elastomer im "Basisgel" (bei Methode A) | | 24 | 24 | 24 |
| Gew.-% Elastomer im fertigen Gel | | 20 | 18 | 14 |
| Verwendete Vorschrift | | A | A | A |

| | | | | |
|---|---|---|---|---|
| ¹PSF-2cSt Pure Silicone Fluid Dodecamethylpentasiloxane zu beziehen bei CLEARCO PRODUCTS CO. INC., U.S.A.; Viskosität bei 25°C; ²Verhältnis M / M^{Vi} / Q = 7,6 / 1 / 11,4, Mₙ = 2570, M_{w} = 5440, Jodzahl = 18; ³Polysiloxan mit 3-Mercaptopropylgruppen; Viskosität 190 mm²/s bei 25 °C, Mercaptangehalt 0,29 Gew.-%; ⁴WACKER® CATALYST OL zu beziehen bei Wacker Chemie AG; | | | | |

**Beispiele 8** - **14** sind Beispiele für Gele, bei denen ein Vernetzer mit einem sehr niedrigen Gehalt an Si-H Gruppen verwendet wird, als einziger Vernetzer oder in Kombination mit einem weiteren Vernetzer. Als Verdünnungsmittel wurde nichtflüchtiges, lineares Polydimethylsiloxan (5 mm²/s bei 25°C) gewählt. Es werden lagerstabile, cremige Gele erhalten, welche für die Verwendung in kosmetischen Formulierungen geeignet sind und signifikante Mengen hydrophiler Flüssigkeit aufnehmen können.

Die Vergleichsbeispiele V4, V5 und V6 zeigen Gele im gleichen Verdünnungsmittel, wie es in den Beispielen 8 - 14 verwendet wurde. Vergleichsbeispiel V4 enthält jedoch ausschließlich einen Vernetzer mit sehr hohem Gehalt an Si-H-Gruppen, wie in EP 1 132 430 A1 offenbart. Man erhält ein niederviskoses, fließendes Gel mit öligem Gefühl, welches für die beschriebenen Anwendungen nicht geeignet ist. Vergleichsbeispiel V5 enthält einen Vernetzer mit sehr hohem Gehalt an Si-gebundenen Wasserstoff in Kombination mit 25 Gew.-% eines weniger funktionellen Vernetzers. Man erhält ein niederviskoses, fließendes Gel mit öligem Gefühl, welches für die beschriebenen Anwendungen nicht geeignet ist.

Beispiel V6 hat eine zum Beispiel 10 ähnliche Rezeptur, wurde jedoch ohne die erfindungsgemäße Verbindung (1b), also ohne eine Verbindung, die einen Glycosidrest und eine hydrosilylierbare Endgruppe aufweist, hergestellt. Das Gel ist cremig, lagerstabil und transparent, nimmt jedoch weder Wasser noch Glycerin auf, wie die Vergleichsbeispiele V8 (Tabelle 7) und V12 (Tabelle 8) zeigen.

**TABELLE 4 - ELASTOMERGEL FORMULIERUNGEN:**

| Beispiel: | | V4 | V5 | 8 | 9 | 10 | 11 |
|---|---|---|---|---|---|---|---|
| Verdünnungsmittel (g) | Polydimethyl -siloxan (5 mm²/s)¹ | 760 | 770 | 760 | 760 | 760 | 770 |
| ungesättigtes Silikonharz² (g) | | 206,6 | 506 | 157,4 | 118,4 | 70,4 | 118 |
| Si-H-haltiger Vernetzer (g) | Nr. 1 (0,46 % H) | 23 | 21,2 | | | | |
| | Nr. 2 (0,14 % H) | | 5,3 | 58,9 | 32,4 | | 32,4 |
| | Nr. 3 (0,026 % H) | | | 14,3 | 78,6 | 159,2 | 78,6 |
| (2-Allyloxy-ethoxy)-glucosid (g) | | 5 | 5 | 5 | 5 | 5 | 8 |
| 1,2-Propandiol (g) | | 5 | 5 | 5 | 5 | 5 | 8 |
| Platingift (g) | Mercaptoöl³ | 6,9 | 6,9 | 6,9 | 6,9 | 6,9 | 6,9 |
| Katalysator (Gew.-ppm) | Platin-Komplex⁴ | 5 + 5 | 5 + 5 | 5 + 5 | 5 + 5 | 5 + 5 | 5 + 5 |
| Ansatzgröße (g) | | 999, 6 | 1012,5 | 1000, 6 | 999,4 | 999,6 | 1015,0 |
| Mol Vinyl / Mol Si-H | | 1,4 | 1,41 | 1,43 | 1,49 | 1,61 | 1,59 |
| Viskosität (mPa*s bei 25°C) | | 14400 | 8640 | 106000 | 332000 | 310000 | 133000 |

| Beispiel:¹ | | V4 | V5 | 8 | 9 | 10 | 11 |
|---|---|---|---|---|---|---|---|
| Eigenschaften | | zähflüssig | zähflüssig | cremig, standfest | cremig, standfest | cremig, standfest | cremig, standfest |
| Aussehen | | transluzent | transluzent | transluzent | transluzent | transluzent | transluzent |
| Lagerstabil | | ja | ja | ja | ja | ja | ja |
| Gew.-% Elastomer im "Basisgel" (bei Methode A) | | - | - | - | - | - | - |
| Gew.-% Elastomer im fertigen Gel | | 24 | 24 | 24 | 24 | 24 | 24 |
| Verwendete Vorschrift | | B | B | B | B | B | B |

| | | | | | | | |
|---|---|---|---|---|---|---|---|
| ¹WACKER-BELSIL® DM 5 zu beziehen bei Wacker Chemie AG; Viskosität bei 25°C; ²Verhältnis M / M^{Vi} / Q = 7,6 / 1 / 11,4, Mₙ = 2570, M_{w} = 5440, Jodzahl = 18; ³Polysiloxan mit 3-Mercaptopropylgruppen; Viskosität 190 mm²/s bei 25 °C, Mercaptangehalt 0,29 Gew.-%; ⁴WACKER® CATALYST OL zu beziehen bei Wacker Chemie AG; | | | | | | | |

**TABELLE 5 - ELASTOMERGEL FORMULIERUNGEN:**

| Beispiel: | | 12 | 13 | 14 | V6 |
|---|---|---|---|---|---|
| Verdünnungsmittel (g) | Polydimethylsiloxan (5 mm²/s)¹ | 850 | 960 | 1250 | 810 |
| ungesättigtes Silikonharz² (g) | | 157,4 | 118,4 | 70,4 | 90,3 |
| Si-H-haltiger Vernetzer (g) | Nr. 1 (0,46 % H) | | | | |
| | Nr. 2 (0,14 % H) | 58,9 | 32,4 | | |
| | Nr. 3 (0,026 % H) | 14,3 | 78,6 | 159,2 | 169 |
| (2-Allyloxy-ethoxy) -glucosid (g) | | 5 | 5 | 5 | - |
| 1,2-Propandiol (g) | | 5 | 5 | 5 | - |
| Platingift (g) | Mercaptoöl³ | 6,9 | 6,9 | 6,9 | 3, 8 |
| Katalysator (Gew.-ppm) | Platin-Komplex⁴ | 5 + 5 | 5 + 5 | 5 + 5 | 5 |
| Ansatzgröße (g) | | 1197,5 | 1496,3 | 1006,5 | 1073,1 |
| Mol Vinyl / Mol Si-H | | 1,43 | 1,49 | 1,61 | 1,58 |
| Viskosität (mPa*s bei 25°C) | | 60000 | 88100 | 98000 | 443000 |
| Eigenschaften | | cremig, standfest | cremig, standfest | cremig, standfest | cremig, standfest |
| Aussehen | | transluzent | transluzent | transluzent | transpa rent |
| Lagerstabil | | ja | ja | ja | ja |
| Gew.-% Elastomer im "Basisgel" (bei Methode A) | | 24 | 24 | 24 | - |
| Gew.-% Elastomer im fertigen Gel | | 22 | 20 | 16 | 24 |
| Verwendete Vorschrift | | A | A | A | B |

| | | | | | |
|---|---|---|---|---|---|
| ¹WACKER-BELSIL® DM 5 zu beziehen bei Wacker Chemie AG; Viskosität bei 25°C; ²Verhältnis M / M^{Vi} / Q = 7, 6 / 1 / 11,4, Mₙ = 2570, M_{w} = 5440, Jodzahl = 18; ³Polysiloxan mit 3-Mercaptopropylgruppen; Viskosität 190 mm²/s bei 25 °C, Mercaptangehalt 0,29 Gew.-%; ⁴WACKER® CATALYST OL zu beziehen bei Wacker Chemie AG; | | | | | |

### Beispiele 15 - 21 und Vergleichsbeispiel V7 - V8: Wasserhaltige Mischungen

Die erfindungsgemäßen Organopolysiloxangele gemäß den Beispielen 1 - 4 und 10, sowie die Gele aus den Vergleichsbeispielen V3 und V6 wurden zur Herstellung von wasserhaltigen Mischungen eingesetzt. Hierfür wurden die Gele mit variablen Mengen Wasser versetzt. Das Wasser wurde mit einem Ultra-Turrax®-Mischer bei 1000 U/min und Raumtemperatur portionsweise in das Gel eingeschert. Die Zusammensetzungen und Ergebnisse sind in den Tabellen 6 und 7 aufgeführt. Die Beispiele 15, 16, 18, 20 und 21 zeigen 1-phasige, homogene, stabile, cremige, transluzente Mischungen aus erfindungsgemäßen Organopolysiloxangel und Wasser. Die Beispiele 17 und 19 zeigen gesättigte Mischungen. Sie sind stabil, transluzent und cremig, aber die Oberfläche ist mit einzelnen, feinen Tröpfchen besetzt. Das ist ein Anzeichen dafür, dass das Netzwerk der erfindungsgemäßen Organopolysiloxangele mit Lösemittel gesättigt sind. Das Gel kann jetzt kein zusätzliches Wasser mehr aufnehmen. Bei den Vergleichsbeispielen V3 und V6 handelt es sich um cremige Organopolysiloxangele, welche ohne Mitverwendung der erfindungsgemäßen Verbindung (1b), also ohne eine Verbindung, die einen Glycosidrest und eine hydrosilylierbare Endgruppe aufweist, hergestellt worden sind. Sie nehmen kein Wasser auf und trennen sich in zwei Phasen, eine klare Wasserphase und eine klare Gelphase (Vergleichsbeispiele V7 und V8).

**TABELLE 6 - Wässrige Mischungen:**

| | | | | | |
|---|---|---|---|---|---|
| Beispiel: | 15 | 16 | 17 | 18 | 19 |
| Gel aus Beispiel | 1 | 2 | 2 | 3 | 3 |
| Wasser Gew.-%¹ | 11 | 11 | 18 | 11 | 25 |
| Gesamtmasse (g) | 100 | 100 | 100 | 100 | 100 |
| Mischung gebildet | ja | ja | ja | ja | ja |
| Viskosität (mPa*s bei 25°C) | 171000 | 119000 | - | 271000 | - |
| Aussehen | cremig, transluzent, 1-phasig, homogen | cremig, transluzent, 1-phasig, homogen | cremig, transluzent, 1-phasig, homogen² | cremig, transluzent, 1-phasig, homogen | cremig, transluzent, 1-phasig, homogen² |

| | | | | | |
|---|---|---|---|---|---|
| ¹ Gew.-% Wasser bezogen auf das Gesamtgewicht des Organopolysiloxangels. ² Die Bildung feiner Tröpfchen weist auf das Erreichen der Sättigungsgrenze hin. | | | | | |

**TABELLE 7 Wässrige Mischungen:**

| | | | | |
|---|---|---|---|---|
| Beispiel: | 20 | V7 | 21 | V8 |
| Gel aus Beispiel | 4 | V3 | 10 | V6 |
| Wasser Gew.-%¹ | 43 | 10 | 18 | 10 |
| Gesamtmasse (g) | 100 | 100 | 100 | 100 |
| Mischung gebildet | ja | nein | ja | nein |
| Viskosität (mPa*s bei 25°C) | 55900 | - | 330000 | - |
| Aussehen | cremig, transluzent, 1-phasig, homogen | 2-phasig, Wasserphase klar | cremig, transluzent, 1-phasig, homogen | 2-phasig, Wasserphase klar |

| | | | | |
|---|---|---|---|---|
| ¹ Gew.-% Wasser bezogen auf das Gesamtgewicht des Organopolysiloxangels. | | | | |

### Beispiele 22 - 27 und Vergleichsbeispiele V9 und V10: Glycerin-haltige Mischungen

Die erfindungsgemäßen Organopolysiloxangele gemäß den Beispielen 1 - 4, 10 und 11 und die Gele aus den Vergleichsbeispiele V6 wurden zur Herstellung von Glycerinhaltigen Mischungen eingesetzt. Hierfür wurden die Gele mit variablen Mengen Glycerin versetzt. Das Glycerin wurde mit einem Ultra-Turrax®-Mischer bei 1000 U/min und Raumtemperatur portionsweise in das Gel eingeschert. Die Zusammensetzungen und Ergebnisse sind in Tabelle 8 aufgeführt.

Die Beispiele 22 - 27 zeigen 1-phasige, homogene, stabile, cremige, weiße Mischungen aus erfindungsgemäßen Organopolysiloxangel und Glycerin. Vergleichsbeispiele V3 und V6 sind cremige Organopolysiloxangele, welche ohne Mitverwendung der erfindungsgemäßen Verbindung (1b), also ohne eine Verbindung, die einen Glycosidrest und eine hydrosilylierbare Endgruppe aufweist, hergestellt worden sind. Sie nehmen zunächst sehr geringfügige Mengen Glycerin auf, d.h. weniger als 5 Gew.-%, und trennen sich dann in zwei Phasen (Vergleichsbeispiele V12 und V13).

**TABELLE 8 - Mischungen mit Glycerin:**

| | | | | | | | | |
|---|---|---|---|---|---|---|---|---|
| Beispiel: | 22 | 23 | 24 | 25 | 26 | 27 | V12 | V13 |
| Gel aus Beispiel | 1 | 2 | 3 | 4 | 10 | 11 | V6 | V3 |
| Glycerin Gew.-%¹ | 67 | 100 | 37 | 100 | 43 | 100 | 20 | 20 |
| Gesamtmasse (g) | 100 | 100 | 100 | 100 | 100 | 100 | 100 | 100 |
| Mischung gebildet | ja | ja | ja | ja | ja | ja | Nein | Nein |
| Viskosität (mPa*s bei 25°C) | 295000 | 323000 | 358000 | 104000 | 597000 | 310000 | - | - |
| Aussehen | 1-phasig, homogen, cremig, weiß | 1-phasig, homogen, cremig, weiß | 1-phasig, homogen, cremig, weiß | 1-phasig, homogen, cremig, weiß | 1-phasig, homogen, cremig, weiß | 1-phasig, homogen, cremig, weiß | 2-phasig | 2-phasig |

| | | | | | | | | |
|---|---|---|---|---|---|---|---|---|
| ¹ Gew.-% Glycerin bezogen auf das Gesamtgewicht des Organopolysiloxangels. | | | | | | | | |

Die erfindungsgemäßen Organopolysiloxangele eignen sich hervorragend für die Herstellung unterschiedlicher Kosmetikprodukte:

### Beispiel 28: Hair-Mask-Conditioner

Ein Hair-Mask-Conditioner wurde unter Verwendung von Beispiel 14 nach folgendem Verfahren hergestellt (unter Teile sind im Folgenden Gewichtsteile zu verstehen):
Wasser wird vorgelegt und unter Rühren 1,5 Teile Hydroxyethylcellulose zugegeben. Anschließend wird 1 Teil PEG-40 Hydrogenated Castor Oil gelöst und 2 Teile Beispiel 14 zugegeben. Diese Mischung wird auf 75°C aufgeheizt. Während des Aufheizens werden 1,5 Teile Cetyl Alcohol, 3 Teile Stearyl Alcohol, 1 Teil Stearamidopropyl Dimethylamine, 3 Teile Behentrimonium Chloride, 2 Teile Glycerin und 1 Teil Simmondsia Chinensis (Jojoba) Seed Oil zugegeben. Die Mischung wird gerührt bis 75°C erreicht sind und die Inhaltsstoffe gelöst vorliegen. Dann wird die Mischung abgekühlt. Bei 40°C werden 0,2 Teile Citric Acid, 1 Teil Ethylhexyl Methoxycinnamate, 0,1 Teil Vitis Vinifera (Grape) Seed Oil und 0,1 Teil Panthenol zugegeben. Anschließend wird die Mischung mit 0,1 Teil Methylchloroisothiazolinone, Methylisothiazolinone konserviert. Die Formulierung wird 5 Minuten unter Rühren homogenisiert.

**TABELLE 9 - Bestandteile Hair-Mask-Conditioner**

| **Phase** | **Handelsname** | **INCI-Name** | **Teile** |
|---|---|---|---|
| A | Cremophor RH 40 ¹⁾ | PEG-40 Hydrogenated Castor Oil | 1,00 |
| A | Beispiel 14 | | 2,00 |
| A | Natrosol 250 HHR ²⁾ | Hydroxyethylcellulose | 1,50 |
| A | Wasser | Aqua (DI Water) | 82,50 |
| B | Cetylalkohol ³⁾ | Cetyl Alcohol | 1,50 |
| B | Genamin KDMP ⁴⁾ | Behentrimonium Chloride | 3,00 |
| B | Glycerin ⁵⁾ | Glycerin | 2,00 |
| B | Incromine® SD-PA-(MH) ⁶⁾ | Stearamidopropyl Dimethylamine | 1,00 |
| B | Jojoba Oil Colorless ⁷⁾ | Simmondsia Chinensis (Jojoba) Seed Oil | 1,00 |
| B | Stearylalkohol ⁸⁾ | Stearyl Alcohol | 3,00 |
| C | Zitronensäure ⁹⁾ | Citric Acid | 0,20 |
| C | Escalol 557 ¹⁰⁾ | Ethylhexyl Methoxycinnamate | 1,00 |
| C | Grape Seed Oil ¹¹⁾ | Vitis Vinifera (Grape) Seed Oil | 0,10 |
| C | Panthenol ¹²⁾ | Panthenol | 0,10 |
| D | Kathon CG ¹³⁾ | Methylchloroisothiazolinone, Methylisothiazolinone | 0,10 |

| | | | |
|---|---|---|---|
| Die Rohstoffe sind unter folgenden Herstellern erhältlich: ¹⁾ BASF AG ²⁾ Ashland Inc. ³⁾ Merck KGaA ⁴⁾ Clariant GmbH ⁵⁾ Bernd Kraft GmbH ⁶⁾ Croda GmbH ⁷⁾ Desert Whale Jojoba Co., Inc. ⁸⁾ Merck-Schuchardt ⁹⁾ Sigma ¹⁰⁾ Ashland Inc. ¹¹⁾ Henry Lamotte GmbH ¹²⁾ BASF AG ¹³⁾ Rohm and Haas Company, Inc. | | | |

### Beispiel 29: Hair-Balm

Ein Hair-Balm wurde unter Verwendung von Beispiel 14 nach folgendem Verfahren hergestellt:
Es werden 0,6 Teile Aminomethyl Propanol vorgelegt und 10 Teile BELSIL® P 1101 zugefügt. Unter Rühren werden 27,5 Teile Wasser und 0,4 Teile PEG-40 Hydrogenated Castor Oil zugegeben. Anschließend werden 2 Teile Beispiel 14 zugegeben.

In einem zweiten Gefäß werden 11 Teile Wasser vorgelegt und 0,1 Teil Disodium EDTA unter Rühren gelöst. Dann werden 3 Teile Glycerin und 0,5 Farnesol, Linalool zugegeben. Diese Mischung wird unter Rühren zur Mischung im ersten Gefäß gegeben.

In einem dritten Gefäß werden 43,8 Teile Wasser vorgelegt und 0,7 Teile Acrylates/C10-30 Alkyl Acrylate Crosspolymer gelöst. Dann werden 0,1 Teil Methylchloroisothiazolinone, Methylisothiazolinone und 0,3 Teile Parfum zugegeben. Diese Mischung wird unter Rühren in das erste Gefäß gegeben.

**TABELLE 10 - Bestandteile Hair-Balm**

| **Phase** | **Handelsname** | **INCI-name** | **Teile** |
|---|---|---|---|
| A | AMP-95 ¹⁾ | Aminomethyl Propanol | 0, 62 |
| A | BELSIL® P 1101 | Alcohol, Crotonic Acid/Vinyl C8-12 Isoalkyl Esters/VA/Bis-Vinyldimethicone Crosspolymer | 10,00 |
| B | Cremophor RH 40 ²⁾ | PEG-40 Hydrogenated Castor Oil | 0,40 |
| B | Beispiel 14 | | 2,00 |
| B | Wasser | Aqua (DI Water) | 27,50 |
| C | Glycerin ³⁾ | Glycerin | 3,00 |
| C | Unistab S-69 ⁴⁾ | Farnesol, Linalool | 0,50 |
| C | Versene NA ⁵⁾ | Disodium EDTA | 0,10 |
| C | Wasser | Aqua (DI Water) | 11,00 |
| D | Carbopol Ultrez 21 ⁶⁾ | Acrylates/C10-30 Alkyl Acrylate Crosspolymer | 0,70 |
| D | Kathon CG ⁷⁾ | Methylchloroisothiazolinone , Methylisothiazolinone | 0,05 |
| D | Parfum | Parfum | 0,30 |
| D | Wasser | Aqua (DI Water) | 43,83 |

| | | | |
|---|---|---|---|
| Die Rohstoffe sind unter folgenden Herstellern erhältlich: ¹⁾ Angus Chemical Company ²⁾ BASF AG ³⁾ Bernd Kraft GmbH ⁴⁾ Induchem AG ⁵⁾ Dow Chemical USA ⁶⁾ BF Goodrich Performance Materials ⁷⁾ Rohm and Haas Company, Inc. | | | |

### Beispiel 30: Nourishing Night Cream

Eine Nourishing Night Cream wurde unter Verwendung von Beispiel 13 nach folgendem Verfahren hergestellt:
Phase A und B wurden separat eingewogen und auf 75°C erhitzt. Phase A wurde zu Phase B gegeben und mit einem Ultra Turrax T 25 basic 10 Minuten bei 15000 Umdrehungen pro Minute homogenisiert.

Die Emulsion wurde unter Rühren mit einem Magnetrührer bei 350 UpM auf Raumtemperatur gebracht. Dabei wurden nach dem Unterschreiten einer Temperatur von 40°C die Stoffe der Phase C zugegeben und eingerührt.

Als letzte Komponente wurde Beispiel 13 zunächst mit einer Spatel in die Emulsion gerührt. Danach wird die Mischung erneut mit dem Ultra Turrax homogenisiert, 3 Minuten bei 11000 Umdrehungen pro Minute.

**Tabelle 11 - Bestandteile Nourishing Night Cream**

| **Phase** | **INCI** | **Handelsname (Hersteller/Lieferant)** | **Teile** |
|---|---|---|---|
| A | Glycerin | Glycerin 100% wasserfrei (Merck KGaA) | 5,00 |
| A | Aqua (DI Water) | Wasser | 52,42 |
| A | Sodium Chloride | Natriumchlorid (Merck KGaA) | 1,00 |
| B | Diethylhexyl Carbonate | Tegosoft DEC (Evonik Goldschmidt GmbH) | 6,00 |
| B | Argania Spinosa Kernel Oil | Arganoil (Henry Lamotte GmbH) | 1,00 |
| B | Isopropyl Myristate | Isopropylmyristat (Merck-Schuchardt) | 7,00 |
| B | C12-15 Alkyl Benzoate | Tegosoft TN (Goldschmidt Chemical Corporation) | 1,00 |
| B | Coco-Caprylate | Cetiol C5 (Cognis Deutschland GmbH) | 6,00 |
| B | Polyglyceryl-2 Dipolyhydroxystearate | Dehymuls PGPH (Cognis Corporation) | 6,00 |
| B | Vitis Vinifera (Grape) Seed Oil | Grape Seed Oil (A & E Connock Perfumery & Cosmetics) | 3,00 |
| B | Aloe Barbadensis Leaf Juice | TN001 Aloe Vera Powder (Terry Laboratories, Inc.) | 0,03 |
| B | Magnesium Stearate | Magnesiumstearat (Riedel de Haen) | 2,00 |
| B | Cera Alba | Bienenwachs (Fluka Chemie AG) | 3,00 |
| C | Parfum | Parfum 671 217 C Amor Girl (Fragrance Recources) | 0,25 |
| C | Phenoxyethanol, Methylparaben, | Euxyl K 300 (Schülke & Mayr) | 0,30 |
| | Butylparaben, Ethylparaben, Propylparaben, Isobutylparaben | | |
| D | | Beispiel 13 | 6,00 |

### Beispiel 31: Satin Liquid Foundation SPF 10

Eine Satin Liquid Foundation SPF 10 wurde unter Verwendung von Beispiel 14 nach folgendem Verfahren hergestellt:
Die Öle der Phase A werden eingewogen und verrührt. Das Harz wird mit einem Magnetrührer bei 350 UpM eingerührt bis es sich gelöst hat. Die Stoffe der Phase B werden zu Phase A gewogen und unter Rühren auf 75°C aufgeheizt. Die Wasserphase D wird unter Rühren auf 75°C geheizt. Die Pigmente und Komponenten der Phase C werden mit einem Spatel vermischt und dann unter Rühren zu Phase D gegeben. Die Mischung wird auf 75°C gehalten.

In einem großen Becherglas wird die Phase C+D mit der Phase A+B bei 75°C mit einem Ultra Turrax T 25 basic 10 Minuten bei 15000 Umdrehungen pro Minute homogenisiert. Dabei wird auf die homogene Verteilung der Pigmente geachtet.

Die Emulsion wurde unter Rühren mit einem Magnetrührer bei 350 UpM auf Raumtemperatur gebracht. Dabei wurden nach dem Unterschreiten einer Temperatur von 40°C die Stoffe der Phase E zugegeben und eingerührt.

Als letzte Komponente wurde Beispiel 14 zunächst mit einer Spatel in die Emulsion gerührt. Danach wird die Mischung erneut mit dem Ultra Turrax homogenisiert, 3 Minuten bei 11000 Umdrehungen pro Minute.

**TABELLE 12 - Bestandteile Satin Liquid Foundation SPF 10**

| **Phase** | **INCI** | **Handelsname (Hersteller/Lieferant)** | **Teile** |
|---|---|---|---|
| A | PPG-2 Myristyl Ether Propionate | Crodamol PMP (Croda GmbH) | 0,80 |
| A | Ethylhexyl Salicylate | Eusolex OS (Merck KGaA) | 4,80 |
| A | Trimethylsiloxysilicate | BELSIL® TMS 803 (Wacker Chemie AG) | 1, 90 |
| A | Caprylic/Capric Triglyceride | Miglyol 812 N (Sasol Germany GmbH) | 1, 90 |
| A | Isopropyl Myristate | Isopropylmyristat (Merck-Schuchardt) | 5,00 |
| A | Octyldodecyl Neopentanoate | Elefac I-205 (Alzo International Inc.) | 1,00 |
| B | C26-28 Alkyl Dimethicone | BELSIL® CDM 3526 VP (Wacker Chemie AG) | 1, 90 |
| B | Glyceryl Stearate SE | Tegin (Evonik Goldschmidt GmbH) | 1,90 |
| B | Butyl Methoxydibenzoylmethane | Eusolex 9020 (Merck KGaA) | 1,90 |
| B | Sorbitan Trioleate | Span 85 (Uniqema) | 1,90 |
| C | CI 77891 | Unipure White LC 981 (LCW) | 3,91 |
| C | CI 77492 | Unipure Yellow LC 181 (LCW) | 0,51 |
| C | Aluminum Starch Octenylsuccinate | Covafluid AMD (LCW) | 0,90 |
| C | Titanium Dioxide, Alumina, Simethicone | Eusolex T 2000 (Merck KGaA) | 2,90 |
| C | Magnesium Aluminum | Magnabrite S | 0,46 |
| | Silicate | (AMCOL Health & Beauty Solution) | |
| C | CI 77499 | Unipure Black LC 989 (LCW) | 0,03 |
| C | CI 77491+CI 77492+CI 77499 | Unipure Brown LC 887 (LCW) | 0,11 |
| C | CI 77491 | Unipure Red LC 383 (LCW) | 0,17 |
| C | Boron Nitride | Tres BN PUHP1109 (Saint-Gobain Advanced Ceramics Boron) | 0,10 |
| C | Talc | Luzenac Pharma UM (Luzenac Europe SAS) | 1, 90 |
| D | Aqua (DI Water) | Water | 48,76 |
| D | Butylene Glycol | 1,3 Butandiol | 2,90 |
| D | Glycerin | Glycerin 100% waterless (Merck KGaA) | 2, 90 |
| D | Polysorbate 60 | Tween 60. (Merck KGaA) | 1, 90 |
| D | Tetrasodium EDTA | EDETA B Pulver (BASF Corporation) | 0,30 |
| D | Xanthan Gum | Keltrol SF (CP Kelco) | 0,50 |
| E | Tocopheryl Acetate | Copherol 1250 (Cognis Corporation) | 0,30 |
| E | Parfum | Parfum SCE 243993 Pitanga (Scentec) | 0,25 |
| E | Phenoxyethanol, Methylparaben, Butylparaben, Ethylparaben, Propylparaben, Isobutylparaben | Euxyl K 300 (Schülke & Mayr) | 0,50 |
| F | | Beispiel 14 | 7,70 |

### Beispiel 32: Anhydrous Sun Gel SPF 20

Eine Anhydrous Sun Gel SPF 20 Formulierung wurde unter Verwendung von Beispiel 14 nach folgendem Verfahren hergestellt:
Phase A wird mit einem Magnetrührer 10 Minuten bei 350 UpM gerührt und auf 75°C erhitzt bis alle Komponenten geschmolzen sind. Die Phase B wird mit Phase A unter Rühren mit einem Dissolver bei 2000 Umdrehungen pro Minute für 15 Minuten homogenisiert.

Die Formulierung wurde unter Rühren mit dem Dissolver auf Raumtemperatur abgekühlt. Dabei wurden nach dem Unterschreiten einer Temperatur von 40°C die Stoffe der Phase C zugegeben und und bei 1000 Umdrehungen pro Minute eingerührt.

**TABELLE 13 - Bestandteile Anhydrous Sun Gel SPF 20**

| **Phase** | **INCI** | **Handelsname (Hersteller/Lieferant)** | **Teile** |
|---|---|---|---|
| A | Caprylyl Methicone | Silcare Silicone 41M15 (Clariant GmbH) | 0,30 |
| A | Ethylhexyl Salicylate | Eusolex OS (Merck KGaA) | 5,00 |
| A | Polymethylsilsesquioxane | BELSIL® PMS MK Powder (Wacker Chemie AG) | 0,70 |
| A | Diisobutyl Adipate | Crodamol Diba (Croda GmbH) | 0,70 |
| A | Octocrylene | Eusolex OCR (Merck KGaA) | 3,00 |
| A | Sorbitan Olivate | Olivem 900 (Quantiq) | 16,00 |
| A | Butyl Methoxydibenzoylmethane | Eusolex 9020 (Merck KGaA) | 1,50 |
| A | Trimethylsiloxysilicate | BELSIL® TMS 803 (Wacker Chemie AG) | 4,00 |
| A | Ethylhexyl Methoxycinnamate | Escalol 557 (Ashland Inc.) | 7,50 |
| B | Silica Dimethyl Silylate | HDK® H15 (Wacker Chemie AG) | 1,30 |
| C | Cyclopentasiloxane | Cyclopentasiloxane (Wacker Chemie AG) | 6,30 |
| C | | Beispiel 14 | 26,00 |
| C | Disiloxane | BELSIL® DM 0.65 (Wacker Chemie AG) | 4,80 |
| C | Parfum | Delight 71 028 (Fragrance Recources) | 0,20 |
| C | Dimethicone/Vinyltrimethyl siloxysilicate Crosspolymer | BELSIL® RG 100 (Wacker Chemie AG) | 22,70 |

### Beispiel 33: Tropical Summer Butter

Eine Tropical Summer Butter wurde unter Verwendung von Beispiel 14 nach folgendem Verfahren hergestellt:
Zur Herstellung von Phase A wurden Wasser und Propandiol in einem großen Becherglas gemischt. Danach wurde langsam unter Rühren mit einem Magnetrührer Xanthan Gum zugegeben und auf 80-85 °C geheizt. Die Bestandteile der Phase B wurden in einem Becherglas mit einem Magnetrührer gemischt und auf 80-85 °C geheizt. Die Phase B wurde langsam zur Phase A gegeben und mit einem Ultra Turrax T 25 basic bei 13000 UpM 10 Minuten homogenisiert.

Die Formulierung wurde unter langsamem Rühren mit einem Magnetrührer bei 350 UpM auf Raumtemperatur abgekühlt. Dabei wurden nach dem Unterschreiten einer Temperatur von 40°C die Stoffe der Phase C nacheinander zugegeben. Beispiel 14 wurde als letzte Komponente zugegeben. Danach wurde 3 Minuten mit einem Ultra Turrax T 25 basic bei 11000 UpM homogenisiert.

**TABELLE 14 - Bestandteile Tropical Summer Butter**

| **Phase** | **INCI** | **Handelsname (Hersteller/Lieferant)** | **Teile** |
|---|---|---|---|
| A | Aqua (DI Water) | Wasser | 51,35 |
| A | Xanthan Gum | Keltrol CG-SFT (CP Kelco) | 0,20 |
| A | Propylene, Glycol | Propandiol 1.2 (BASF Corporation) | 3,00 |
| B | Butyrospermum Parkii (Shea Butter) | Cetiol SB 45 (Cognis Corporation) | 3,50 |
| B | Olea Europaea (Olive) Fruit Oil | Olivenöl (Henry Lamotte GmbH) | 5,00 |
| B | Persea Gratissima (Avocado) Oil | Avocadoöl (Henry Lamotte GmbH) | 4,00 |
| B | PPG-15 Stearyl Ether | Cetiol E (BASF Corporation) | 2,50 |
| B | Cetyl Alcohol | Cetylalkohol (Merck KGaA) | 3,50 |
| B | Ozokerite | Ozokerit | 1,00 |
| B | Steareth-2 | Eumulgin S2 (BASF Corporation) | 3,00 |
| B | C26-28 Alkyl Methicone | BELSIL® CM 7026 VP (Wacker Chemie AG) | 2,00 |
| B | Dimethicone | BELSIL® DM 10 (Wacker Chemie AG) | 4,00 |
| B | Dicaprylyl Ether | Cetiol OE (Cognis Corporation) | 3,00 |
| B | BHT | Ionol CP (Oxiris Chemicals S.A) | 0,05 |
| B | Cetearyl Olivate, Sorbitan Olivate | Olivem 1000 (Quantiq) | 3,00 |
| B | Isopropyl Myristate | Isopropylmyristat (Merck-Schuchardt) | 2,00 |
| B | Ceteareth-20 | Eumulgin B2 | 2,10 |
| | | (Cognis Corporation) | |
| C | Phenoxyethanol, Methylparaben, Butylparaben, Ethylparaben, Propylparaben, Isobutylparaben | Euxyl K 300 (Schülke & Mayr) | 0,30 |
| C | Disiloxane | BELSIL® DM 0.65 (Wacker Chemie AG) | 2,00 |
| C | Parfum | Parfum Mangoo (Fragrance Recources) | 0,50 |
| D | | Beispiel 14 | 4,00 |

### Beispiel 34: BB Cream

Eine BB Cream wurde unter Verwendung von Beispiel 14 nach folgendem Verfahren hergestellt:
Die öligen Bestandteile der Phase A wurden mit einem Magnetrührer bei 350 UpM gemischt. Dann wurde zunächst BELSIL® TMS 803 und bis zum vollständigen Auflösen weiter gerührt. Anschließend wurden die restlichen Komponenten der Phase A zugegeben. Danach wurden die Bestandteile der Phase B zugegeben und auf 75 °C geheizt. Die Bestandteile von Phase C wurden gesondert gemischt, auf 75 °C geheizt und langsam zur Phase AB gegeben. Danach wurde mit einem Ultra Turrax T 25 basic 13 bei 15000 UpM homogenisiert. Die Mischung wird unter Rühren mit einem Magnetrührer bei 350 UpM auf Raumtemperatur gekühlt. Dabei wurden nach dem Unterschreiten einer Temperatur von 40°C die Stoffe der Phase D nacheinander zugegeben. Beispiel 14 wurde als letzte Komponente zugegeben. Danach wurde 3 Minuten mit einem Ultra Turrax T 25 basic bei 11000 UpM homogenisiert.

**TABELLE 15 - Bestandteile BB Cream**

| **Phase** | **INCI** | **Handelsname (Hersteller/Lieferant)** | **Teile** |
|---|---|---|---|
| A | Panthenol | D-Panthenol USP (BASF AG) | 1,00 |
| A | Butyl Methoxydibenzoylmethane | Eusolex 9020 (Merck KGaA) | 3,00 |
| A | Ethylhexyl Salicylate | Eusolex OS (Merck KGaA) | 4,00 |
| A | Polyglyceryl-2 Dipolyhydroxystearate | Dehymuls PGPH (Cognis Corporation) | 4,00 |
| A | Isododecane, Disteardimonium, Hectorite | Bentone Gel ISDV (Elementis Specialties) | 5,00 |
| A | Trimethylsiloxysilicate | BELSIL® TMS 803 (Wacker Chemie AG) | 2,00 |
| A | Cyclopentasiloxane, Caprylyl Dimethicone Ethoxy Glucoside | BELSIL® SPG 128 VP (Wacker Chemie AG) | 5,00 |
| A | Octocrylene | Eusolex OCR (Merck KGaA) | 10,00 |
| A | Aloe Barbadensis Leaf Juice | TN001 Aloe Vera Powder 200* Conc. (Terry Laboratories, Inc.) | 0,03 |
| A | Isopropyl Myristate | Isopropylmyristat (Merck-Schuchardt) | 4,00 |
| B | Talc, CI 77891, CI 77492, Hydrogen Dimethicone, Aluminum Hydroxide | FDP-C-Yellow3 (Prodotti Gianni S.p.A) | 0, 60 |
| B | Talc, CI 77891, CI 77499, Hydrogen Dimethicone, Aluminum | FDP-C-Black3 (Prodotti Gianni S.p.A) | 0,15 |
| | Hydroxide | | |
| B | Talc | Talc Superiore M10 DEC (Imerys Talc) | 6,00 |
| B | Talc, CI 77891, CI 77491, Hydrogen Dimethicone, Aluminum Hydroxide | FDP-C-Red3 (Prodotti Gianni S.p.A) | 0,20 |
| B | Titanium Dioxide, Alumina, Simethicone | Eusolex T 2000 (Merck KGaA) | 5,00 |
| B | Nylon-12 (and) sodium hyaluronate | Orgasol Hydra + (Arkema) | 1,00 |
| B | Boron Nitride | Tres BN PUHP500 (Saint-Gobain Advanced Ceramics Boron) | 1,00 |
| C | Glycerin | Glycerin (Bernd Kraft GmbH) | 2,50 |
| C | Sodium Chloride | Natriumchlorid (Merck KGaA) | 1,00 |
| C | Aqua (DI Water) | Wasser | 32,02 |
| D | Phenoxyethanol, Methylparaben, Butylparaben, Ethylparaben, Propylparaben, Isobutylparaben | Euxyl K 300 (Schülke & Mayr) | 0,30 |
| D | Parfum | Parfum SCE 243993 Pitanga (Scentec) | 0,20 |
| D | Disiloxane | BELSIL® DM 0.65 (Wacker Chemie AG) | 6,00 |
| D | Glycerin (and) Water (and) Alcohol (and) Leontopoidum Alpinum Extract | Alpaflor Edelweiss B. (DSM Nutritional Products AG) | 1,00 |
| E | | Beispiel 14 | 5,00 |

### Beispiele 35-37: Skin-Care Lotionen enthaltend Beispiele 5-7

Es wurden drei Skin-Care Lotionen unter Verwendung von Beispiel 5, 6 oder 7 nach folgendem Verfahren hergestellt:
Phase A wird unter Rühren auf 75°C geheizt. Phase B wird unter Rühren auf 50°C geheizt. Man lässt langsam Xanthan Gum in Phase B einrieseln. Danach wird Phase B stark gerührt, bis die Phase homogen ist und weiter auf 75°C geheizt. Phase A wird langsam zu Phase B gegeben, während mit einem Ultra Turrax T 25 basic bei 13 UpM homogenisiert wird. Die Emulsion wird auf Raumtemperatur abgekühlt und die Inhaltsstoffe der Phase C eingerührt. Das Elastomergel der Phase C wird mit dem Ultra Turrax T 25 basic für 3 Minuten bei 11000 UpM in die fertige Mischung eingearbeitet.

**TABELLE 16 - Bestandteile Skin-Care Lotion**

| **Phase** | **Handelsname** (Hersteller/ Lieferant) | **INCI-Name** | **Beispiel** | | |
|---|---|---|---|---|---|
| | | | **35** | **36** | **37** |
| A | Emulgade SE-PF (Cognis Corporation) | Glyceryl Stearate, Ceteareth-20, Ceteareth-12, Cetearyl Alcohol, Cetyl Palmitate | 5,00 | 5,00 | 5,00 |
| A | Isopropylmyristat (Merck-Schuchardt) | Isopropyl Myristate | 4,00 | 4,00 | 4,00 |
| A | Lanette O (Cognis Corporation) | Cetearyl Alcohol | 1,00 | 1,00 | 1,00 |
| A | Myritol 331 (Cognis Deutschland GmbH) | Cocoglycerides | 3,75 | 3,75 | 3,75 |
| A | Tegosoft DEC (Evonik Goldschmidt GmbH) | Diethylhexyl Carbonate | 3,25 | 3,25 | 3,25 |
| B | Dekaben MEP (IMCD Deutschland GmbH & Co. KG) | Phenoxyethanol, Ethylparaben, Methylparaben, Propylparaben | 0,50 | 0,50 | 0,50 |
| B | Glycerin (Bernd Kraft GmbH) | Glycerin | 3,00 | 3,00 | 3,00 |
| B | Keltrol CG-SFT (CP Kelco) | Xanthan Gum | 0,50 | 0,50 | 0,50 |
| B | Wasser | Aqua (DI Water) | 70,80 | 70,80 | 70,80 |
| C | Beispiel 5 | | 8,00 | | |
| C | Beispiel 6 | | | 8,00 | |
| C | Beispiel 7 | | | | 8,00 |
| C | Parfum 671 217 C Amor Girl (Fragrance Recources) | Parfum | 0,20 | 0,20 | 0,20 |

### Beispiele 38 und 39: Skin-Care Lotionen enthaltend Beispiele 8 und 13

Es wurden zwei Skin-Care Lotionen unter Verwendung von Beispiel 8 oder 13 nach folgendem Verfahren hergestellt:
Phase A wird unter Rühren auf 75°C geheizt. Phase B wird unter Rühren auf 50°C geheizt. Man lässt langsam Xanthan Gum in Phase B einrieseln. Danach wird Phase B stark gerührt, bis die Phase homogen ist und weiter auf 75°C geheizt. Phase A wird langsam zu Phase B gegeben, während mit einem Ultra Turrax T 25 basic bei 13 UpM homogenisiert wird. Die Emulsion wird auf Raumtemperatur abgekühlt und die Inhaltsstoffe der Phase C eingerührt. Das Elastomergel der Phase C wird mit dem Ultra Turrax T 25 basic für 3 Minuten bei 11000 UpM in die fertige Mischung eingearbeitet.

**TABELLE 17 - Bestandteile Skin-Care Lotion**

| **Phase** | **Handelsname (Hersteller/Lieferant)** | **INCI-Name** | **Beispiel** | |
|---|---|---|---|---|
| | | | **38** | **39** |
| A | Emulgade SE-PF (Cognis Corporation) | Glyceryl Stearate, Ceteareth-20, Ceteareth-12, Cetearyl Alcohol, Cetyl Palmitate | 5,00 | 5,00 |
| A | Isopropylmyristat (Merck-Schuchardt) | Isopropyl Myristate | 4,00 | 4,00 |
| A | Lanette O (Cognis Corporation) | Cetearyl Alcohol | 1,00 | 1,00 |
| A | Myritol 331 (Cognis Deutschland GmbH) | Cocoglycerides | 3,75 | 3,75 |
| A | Tegosoft DEC (Evonik Goldschmidt GmbH) | Diethylhexyl Carbonate | 3,25 | 3,25 |
| B | Dekaben MEP (IMCD Deutschland GmbH & Co. KG) | Phenoxyethanol, Ethylparaben, Methylparaben, Propylparaben | 0,50 | 0,50 |
| B | Glycerin (Bernd Kraft GmbH) | Glycerin | 3,00 | 3,00 |
| B | Keltrol CG-SFT (CP Kelco) | Xanthan Gum | 0,25 | 0,25 |
| B | Wasser | Aqua (DI Water) | 71,05 | 71,25 |
| C | Beispiel 8 | | 8,00 | |
| C | Beispiel 13 | | | 8,00 |
| C | Parfum 671 217 C Amor Girl (Fragrance Recources) | Parfum | 0,20 | 0,20 |

Organopolysiloxangele bewirken in kosmetischen Anwendungen sensorischen Vorteile, indem sie die Verteilbarkeit des Produkts auf der Haut verbessern und dem Produkt ein seidig sanftes Gefühl verleihen. Als besonders vorteilhaft hat sich hierfür eine Viskosität im Bereich von 75000 - 120000 mPa*s bei 25°C erwiesen. Die Beurteilung der sensorischen Eigenschaften erfolgte durch eine geschulte Gruppe von 5 Probanden.

### Beispiel 40: Sensorische Beurteilung der Organopolysiloxangele aus Beispiel 5, 6 und 13 sowie Vergleichsbeispiel V1 und V4

Die Panelisten haben auf den gereinigten Unterarm auf einer kreisrunden Fläche von 20 cm² jeweils 0,05 g des Produktes aufgetragen und die Organopolysiloxangele im Hinblick auf ihre Verteilbarkeit relativ zueinander verglichen. Das Auftragen erfolgte mit dem Zeigefinger oder Mittelfinger und einer Rotationsgeschwindigkeit von zwei Umdrehungen pro Sekunde. Insgesamt wurden 30 Umdrehungen durchgeführt. Nach einer Wartezeit von 60 Sekunden wurden die Rückstände der Organopolysiloxangele im Hinblick auf ihre Seidigkeit relativ zueinander verglichen.

Die Organopolysiloxangele aus den Beispielen 5 und 6 sowie Vergleichsbeispiel V1 enthalten flüchtiges, lineares Polydimethylsiloxan (2 mm²/s bei 25°C) als Verdünnungsmittel. Das Organopolysiloxangel aus den Beispiel 13 sowie Vergleichsbeispiel V4 enthalten nicht-flüchtiges, lineares Polydimethylsiloxan (5 mm²/s bei 25°C) als Verdünnungsmittel. Da hieraus ein unterschiedliches Verhalten beim Auftragen und beim Rückstand resultiert, wurden die Produkte mit unterschiedlichen Verdünnungsmitteln jeweils getrennt voneinander bewertet.

Nach der Bewertung der Panelisten sind beide Beispiele 5 und 6 sehr gut verteilbar. Die Rückstände beider Beispiele wurden mehrheitlich als seidig eingestuft. Beispiel 6 wurde im Vergleich zu Beispiel 5 in beiden Merkmalen als noch etwas besser eingestuft.
Vergleichsbeispiel V1 konnte den Test nicht regulär durchlaufen, da das Material kein standfestes Gel gebildet hat und aufgrund der ungeeigneten, fließenden Beschaffenheit nach dem Auftragen auf die Haut bereits von alleine verläuft. Vergleichsbeispiel V1 hinterlässt im Gegensatz zu Beispiel 5 und Beispiel 6 keinen matten, seidigen Film, sondern eine ölige, glänzende Schicht, die von keinem der Panelisten als seidig bewertet worden ist.

**TABELLE 18 - Sensorische Bewertung der Organopolysiloxangele aus Beispiel 5 und 6 sowie Vergleichsbeispiel V1**

| **Beispiel** | **Viskosität [mPa*s bei 25°C]** | **Festgehalt [%]** | **Verteilbarkeit** | **Seidigkeit** |
|---|---|---|---|---|
| 5 | 86000 | 20 | + | + |
| 6 | 108000 | 18 | ++ | ++ |
| V1 | 18800 | 24 | -* | -* |

| | | | | |
|---|---|---|---|---|
| * Vergleichsbeispiel V1 konnte nicht regulär getestet werden, da das Produkt keine gelartige Konsistenz besitzt und schon vor dem manuellen Verteilen von alleine auf der Haut verläuft. | | | | |

Nach der Bewertung der Panelisten ist Beispiel 13 sehr gut verteilbar. Der Rückstand wurde mehrheitlich als seidig eingestuft. Vergleichsbeispiel V4 konnte den Test nicht regulär durchlaufen, da das Material kein standfestes Gel gebildet hat und aufgrund der ungeeigneten, fließenden Beschaffenheit nach dem Auftragen auf die Haut bereits von alleine verläuft. Vergleichsbeispiel V4 hinterlässt im Gegensatz zu Beispiel 13 keinen matten, seidigen Film, sondern eine ölige, glänzende Schicht, die von keinem der Panelisten als seidig bewertet worden ist.

**TABELLE 19 - Sensorische Bewertung des Organopolysiloxangels aus Beispiel 13 sowie Vergleichsbeispiel V4**

| **Beispiel** | **Viskosität [mPa*s bei 25°C]** | **Festgehalt [%]** | **Verteilbarkeit** | **Seidigkeit** |
|---|---|---|---|---|
| 13 | 88100 | 20 | ++ | ++ |
| V4 | 14400 | 24 | -* | -* |

| | | | | |
|---|---|---|---|---|
| * Vergleichsbeispiel V4 konnte nicht regulär getestet werden, da das Produkt keine gelartige Konsistenz besitzt und schon vor dem manuellen Verteilen von alleine auf der Haut verläuft. | | | | |

In diesem Vergleichstest zeigt sich ein wesentlicher Vorteil der erfindungsgemäßen Gele, dass sie nämlich bei niedrigem Festgehalt eine hochviskose, cremige Gelstruktur bilden, während die nicht-erfindungsgemäßen Gele aus Vergleichsbeispiel V1, V2, V4 und V5, die auf Basis eines Organopolysiloxans mit einem relativ hohen Gehalt an silicium-gebundenen Wasserstoffatomen hergestellt worden sind, trotz höherem Festgehalt keine gelartige Struktur bilden und von ihren Eigenschaften her einem Öl entsprechen.

### Beispiel 41: Sensorische Beurteilung der Skin-Care Lotionen aus den Beispielen 35, 36, 38 und 39

Wie Beispiel 40 gezeigt hat, eignen sich die nicht-erfindungsgemäßen Vergleichsbeispiele, die auf Basis eines Organopolysiloxans mit einem relativ hohen Gehalt an silicium-gebundenen Wasserstoffatomen hergestellt worden sind (Vergleichsbeispiele V1, V2, V4, und V5), nicht für die Anwendung in kosmetischen Produkten, da sie keine geeignete Konsistenz und keine geeigneten sensorischen Eigenschaften aufweisen. Aus diesem Grund wurden die Materialien in der weiteren Beurteilung der Skin-Care Lotionen nicht berücksichtigt.

Für die Beurteilung der Skin-Care Lotionen aus Beispiel 35, 36, 38 und 39 haben die Panelisten auf den gereinigten Unterarm auf einer kreisrunden Fläche von 20 cm² jeweils 0,05 g des Produktes aufgetragen und die Lotionen im Hinblick auf ihre Verteilbarkeit relativ zueinander verglichen. Nach einer Wartezeit von 60 Sekunden wurden die Rückstände der Organopolysiloxangele im Hinblick auf ihre Seidigkeit relativ zueinander verglichen.

Die Skin-Care Lotionen aus den Beispielen 35 und 36 verwenden Organopolysiloxangele, welche flüchtiges, lineares Polydimethylsiloxan (2 mm²/s bei 25°C) als Verdünnungsmittel enthalten. Die Skin-Care Lotionen aus den Beispielen 38 und 39 verwenden Organopolysiloxangele, welche nicht-flüchtiges, lineares Polydimethylsiloxan (5 mm²/s bei 25°C) als Verdünnungsmittel enthalten. Da hieraus ein unterschiedliches Verhalten beim Auftragen und beim Rückstand resultiert, wurden die Produkte mit unterschiedlichen Verdünnungsmitteln jeweils getrennt voneinander bewertet.

Nach der Bewertung der Panelisten sind beide Beispiele 35 und 36 sehr gut verteilbar. Die Rückstände beider Beispiele wurden mehrheitlich als seidig eingestuft. Beispiel 36 wurde im Vergleich zu Beispiel 35 in der Seidigkeit des Rückstands als noch etwas besser eingestuft.

**TABELLE 20 - Sensorische Bewertung der Skin-Care Lotionen aus den Beispielen 35 und 36**

| **Beispiel** | **enthaltend 8% von Beispiel** | **Verteilbarkeit** | **Seidigkeit** |
|---|---|---|---|
| 35 | 5 | ++ | + |
| 36 | 6 | ++ | ++ |

Nach der Bewertung der Panelisten sind beide Beispiele 38 und 39 sehr gut verteilbar. Die Rückstände beider Beispiele wurden mehrheitlich als seidig eingestuft. Beispiel 39 wurde im Vergleich zu Beispiel 38 in beiden Merkmalen als noch etwas besser eingestuft.

**TABELLE 21 - Sensorische Bewertung der Skin-Care Lotionen aus den Beispielen 38 und 39**

| **Beispiel** | **enthaltend 8% von Beispiel** | **Verteilbarkeit** | **Seidigkeit** |
|---|---|---|---|
| 38 | 8 | + | + |
| 39 | 13 | ++ | ++ |

## Patentansprüche

1. Glycosidreste aufweisende Organopolysiloxangele hergestellt durch Umsetzung von
(la) ungesättigten Organopolysiloxanharzen und
(1b) Verbindungen, die einen Glycosidrest und eine hydrosilylierbare Endgruppe aufweisen,
mit der Maßgabe, dass der gewichtsmäßige Anteil, bezogen auf das Gesamtgewicht des Organopolysiloxangels, 0,1 bis 10 Gew.-%, vorzugsweise 0,1 bis 5 Gew-%, bevorzugt 0,3 bis 2 Gew.-%, ist,
mit
(2) Si-H funktionellen Organopolysiloxanen der allgemeinen Formel
H_{c}R_{3-c}SiO (R₂SiO)ₐ (RHSiO)_{b}SiR_{3-c}H_{c} (I),
wobei
c 0 oder 1, vorzugsweise 0, ist,
R gleich oder verschieden sein kann und einen einwertigen, gegebenenfalls substituierten Kohlenwasserstoffrest mit 1 bis 18 Kohlenstoffatomen je Rest bedeutet,
a und b ganze Zahlen sind, mit der Maßgabe, dass die Summe a+b 66 bis 248, vorzugsweise 98 bis 248, bevorzugt 118 bis 168 ist,
dass die Organopolysiloxane (2) Si-gebundenen Wasserstoff in Mengen von 0,011 bis 0,044 Gew.-%, vorzugsweise von 0,019 bis 0,044 Gew.-%, bevorzugt von 0,022 bis 0,032 Gew.-%, enthalten,
und dass die Anzahl an Si-H-Gruppen pro Molekül durchschnittlich größer 2 und kleiner als 5 ist,
oder Mischungen von (2) Si-H funktionellen Organopolysiloxanen mit
(2') Si-H funktionellen Organopolysiloxanen der allgemeinen Formel
H_{c}R_{3-c}SiO (R₂SiO)ₐ (RHSiO)_{b}SiR_{3-c}H_{c} (I'),
wobei
c 0 oder 1, vorzugsweise 0 ist,
R die oben dafür angegebene Bedeutung hat,
a und b ganze Zahlen sind, mit der Maßgabe, dass die Summe a+b 8 bis 248, vorzugsweise 38 bis 248, ist, und dass die Organopolysiloxane (2') Si-gebundenen Wasserstoff in Mengen von 0,045 bis 0,35 Gew.-%, vorzugsweise von 0,045 bis 0,156 Gew.-%, enthalten,
in Gegenwart von
(3) die Anlagerung von Si-gebundenem Wasserstoff an aliphatische Mehrfachbindung fördernde Katalysatoren,
wobei (la), (1b) und (2) oder Mischungen von (2) und (2') in
(4) Verdünnungsmitteln, vorzugsweise Organopolysiloxanen mit 2 bis 200 Si-Atomen oder organischen Verdünnungsmitteln oder Mischungen von Organopolysiloxanen mit 2 bis 200 Si-Atomen und organischen Verdünnungsmitteln,
dispergiert sind.

2. Organopolysiloxangele nach Anspruch 1, **dadurch gekennzeichnet, dass** nach der Umsetzung durch anschließendes Homogenisieren cremige, lagerstabile Organopolysiloxangele erhalten werden, wobei lagerstabil bedeutet, dass sich die gebildeten Organopolysiloxangele innerhalb von 6 Monaten Lagerung bei Raumtemperatur nicht in zwei oder mehr Phasen entmischen und sich die Weichheit des Gels in diesem Zeitraum nicht wesentlich ändert.

3. Organopolysiloxangele nach Anspruch 2, **dadurch gekennzeichnet, dass** die so erhaltenen Organopolysiloxangele durch Zugabe von weiteren Verdünnungsmitteln (4) und/oder aktiven Wirkstoffen für die Körperpflege oder Gesundheitspflege und ggf. anschließendem Homogenisieren weiter verdünnt werden.

4. Organopolysiloxangele nach Anspruch 1, 2 oder 3, **dadurch gekennzeichnet, dass** als
(1a) ungesättigte Organopolysiloxanharze
MQ-Harze aus Einheiten der Formeln
SiO₂ (Q-Einheiten)
und
R₃SiO_{1/2} und R₂R'SiO_{1/2} (M-Einheiten),
wobei
R gleich oder verschieden sein kann und einen einwertigen, gegebenenfalls substituierten Kohlenwasserstoffrest mit 1 bis 18 Kohlenstoffatomen je Rest bedeutet,
R' einen einwertigen Kohlenwasserstoffrest bedeutet, an den Si-H-Gruppen in einer Hydrosilylierungsreaktion angelagert werden können, vorzugsweise ein ω-Alkenylrest mit 2 bis 12 C-Atomen, bevorzugt ein Vinylrest ist,
mit der Maßgabe, dass die MQ-Harze mindestens 2 Reste R', vorzugsweise mindestens 3 Reste R', enthalten und dass das molare Verhältnis von M-Einheiten zu Q-Einheiten im Bereich von 0,5 bis 4,0, vorzugsweise im Bereich von 0,5 bis 2,0, bevorzugt im Bereich von 0,6 bis 1,5, liegt,
eingesetzt werden.

5. Organopolysiloxangele nach einem der Ansprüche 1 bis 4, **dadurch gekennzeichnet, dass** als Verbindung (1b) solche der allgemeinen Formel
Z-(R¹O)ₚ-R² (III),
wobei
Z einen Glycosidrest bedeutet, der aus 1 bis 10 Monosaccharideinheiten, vorzugsweise Glucoseeinheiten, aufgebaut ist,
R¹ gleich oder verschieden sein kann und einen zweiwertigen Kohlenwasserstoffrest mit 2 bis 4 C-Atomen bedeutet,
R² einen einwertigen Kohlenwasserstoffrest mit 2 bis 12 C-Atomen, an den Si-H-Gruppen in einer Hydrosilylierungsreaktion angelagert werden können, vorzugsweise einen einwertigen eine terminale aliphatische C-C-Mehrfachbindung aufweisenden Kohlenwasserstoffrest mit 2 bis 12 C-Atomen, bevorzugt einen Allylrest, bedeutet, und
p 0 oder eine ganze Zahl von 1 bis 20 ist,
eingesetzt werden.

6. Organopolysiloxangele nach einem der Ansprüche 1 bis 5, **dadurch gekennzeichnet, dass** als Verdünnungsmittel (4) Polydimethylsiloxane mit 2 bis 50 Si-Atomen, aliphatische oder alicyclische Kohlenwasserstoffe mit 4 bis 30 C-Atomen oder Ester von Carbonsäuren mit 2 bis 30 C-Atomen eingesetzt werden.

7. Einphasige, homogene Mischungen enthaltend
(a) Glycosidreste aufweisende Organopolysiloxangele nach einem der Ansprüche 1 bis 6 und
(b) polare oder hydrophile Lösungsmittel.

8. Mischungen nach Anspruch 7, **dadurch gekennzeichnet, dass** die polaren oder hydrophilen Lösungsmittel (b) solche sind ausgewählt sind aus der Gruppe von Wasser, Glycerin, Ethylenglycol, Diethylenglycol, Propylenglycol und deren Gemische.

9. Verfahren zur Herstellung der Glycosidreste aufweisenden Organopolysiloxangele nach einem der Ansprüche 1 bis 6, **dadurch gekennzeichnet, dass**
(1a) ungesättigte Organopolysiloxanharze und
(1b) Verbindungen, die einen Glycosidrest und eine hydrosilylierbare Endgruppe aufweisen, mit der Maßgabe, dass der gewichtsmäßige Anteil, bezogen auf das Gesamtgewicht des Organopolysiloxangels, 0,1 bis 10 Gew.-%, vorzugsweise 0,1 bis 5 Gew-%, bevorzugt 0,3 bis 2 Gew.-%, ist, mit
(2) Si-H funktionellen Organopolysiloxanen der allgemeinen Formel
H_{c}R_{3-c}SiO (R₂SiO)ₐ (RHSiO)_{b}SiR_{3-c}H_{c} (I),
wobei
c 0 oder 1, vorzugsweise 0, ist,
R gleich oder verschieden sein kann und einen einwertigen, gegebenenfalls substituierten Kohlenwasserstoffrest mit 1 bis 18 Kohlenstoffatomen je Rest bedeutet,
a und b ganze Zahlen sind, mit der Maßgabe, dass die Summe a+b 66 bis 248, vorzugsweise 98 bis 248, bevorzugt 118 bis 168 ist,
dass die Organopolysiloxane (2) Si-gebundenen Wasserstoff in Mengen von 0,011 bis 0,044 Gew.-%, vorzugsweise von 0,019 bis 0,044 Gew.-%, bevorzugt von 0,022 bis 0,032 Gew.-%, enthalten,
und dass die Anzahl an Si-H-Gruppen pro Molekül durchschnittlich größer 2 ist,
oder Mischungen von (2) Si-H funktionellen
Organopolysiloxanen mit
(2') Si-H funktionellen Organopolysiloxanen der allgemeinen Formel
H_{c}R_{3-c}SiO (R₂SiO)ₐ (RHSiO)_{b}SiR_{3-c}H_{c} (I'),
wobei
c 0 oder 1, vorzugsweise 0 ist,
R die oben dafür angegebene Bedeutung hat,
a und b ganze Zahlen sind, mit der Maßgabe, dass die Summe a+b 8 bis 248, vorzugsweise 38 bis 248, ist, und dass die Organopolysiloxane (2') Si-gebundenen Wasserstoff in Mengen von 0,045 bis 0,35 Gew.-%, vorzugsweise von 0,045 bis 0,156 Gew.-%, enthalten,
in Gegenwart von
(3) die Anlagerung von Si-gebundenem Wasserstoff an aliphatische Mehrfachbindung fördernde Katalysatoren, umgesetzt werden,
wobei (1a), (1b) und (2) oder Mischungen von (2) und (2') in
(4) Verdünnungsmitteln, vorzugsweise Organopolysiloxanen mit 2 bis 200 Si-Atomen oder organischen Verdünnungsmitteln oder Mischungen von Organopolysiloxanen mit 2 bis 200 Si-Atomen und organischen Verdünnungsmitteln, dispergiert sind.

10. Verfahren nach Anspruch 9, **dadurch gekennzeichnet, dass** die nach der Umsetzung erhaltenen Organopolysiloxangele homogenisiert werden, wobei cremige, lagerstabile Organopolysiloxangele erhalten werden, wobei lagerstabil bedeutet, dass sich die gebildeten Organopolysiloxangele innerhalb von 6 Monaten Lagerung bei Raumtemperatur nicht in zwei oder mehr Phasen entmischen und sich die Weichheit des Gels in diesem Zeitraum nicht wesentlich ändert.

11. Verfahren nach Anspruch 10, **dadurch gekennzeichnet, dass** die so erhaltenen Organopolysiloxangele mit weiteren verdünnungsmittel (4) und/oder aktiven Wirkstoffen für die Körperpflege oder Gesundheitspflege verdünnt werden und ggf. anschließend homogenisiert werden.

12. Verfahren nach Anspruch 9, 10 oder 11, **dadurch gekennzeichnet, dass** als
(la) ungesättigte Organopolysiloxanharze
MQ-Harze aus Einheiten der Formeln
SiO₂ (Q-Einheiten)
und
R₃SiO_{1/2} und R₂R'SiO_{1/2} (M-Einheiten),
wobei R und R' die in Anspruch 4 dafür angegebene Bedeutung haben,
mit der Maßgabe, dass die MQ-Harze mindestens 2 Reste R', vorzugsweise mindestens 3 Reste R', enthalten und dass das molare Verhältnis von M-Einheiten zu Q-Einheiten im Bereich von 0,5 bis 4,0, vorzugsweise im Bereich von 0,5 bis 2,0, bevorzug im Bereich von 0,6 bis 1,5, liegt,
eingesetzt werden.

13. Verfahren nach einem der Ansprüche 9 bis 12, **dadurch gekennzeichnet, dass** als Verbindung (1b) solche der allgemeinen Formel
Z-(R¹O)ₚ-R² (III),
wobei Z, R¹, R² und p die in Anspruch 5 dafür angegebene Bedeutung haben,
eingesetzt werden.

14. Verfahren nach einem der Ansprüche 9 bis 13, **dadurch gekennzeichnet, dass** als Verdünnungsmittel (4) Polydimethylsiloxane mit 2 bis 50 Si-Atomen, aliphatische oder alicyclische Kohlenwasserstoffe mit 4 bis 30 C-Atomen oder Ester von Carbonsäuren mit 2 bis 30 C-Atomen eingesetzt werden.

15. Kosmetische Zusammensetzungen enthaltend Glycosidreste aufweisende Organopolysiloxangele nach einem der Ansprüche 1 bis 6 oder Mischungen nach Anspruch 7 oder 8 oder Organopolysiloxangele hergestellt nach einem der Ansprüche 9 bis 14.

## Claims

1. Organopolysiloxane gels having glycoside residues, prepared by reaction of
(1a) unsaturated organopolysiloxane resins and
(1b) compounds having a glycoside residue and a hydrosilylatable end group,
with the proviso that the proportion by weight, based on the total weight of the organopolysiloxane gel, is 0.1% to 10% by weight, preferably 0.1% to 5% by weight, more preferably 0.3% to 2% by weight,
with
(2) Si-H-functional organopolysiloxanes of the general formula
H_{c}R_{3-c}SiO (R₂SiO)ₐ (RHSiO)_{b}SiR_{3-c}H_{c} (I)
where
c is 0 or 1, preferably 0,
R may be the same or different and is a monovalent, optionally substituted hydrocarbyl radical having 1 to 18 carbon atoms per radical, a and b are integers, with the proviso that the sum of a+b is 66 to 248, preferably 98 to 248, more preferably 118 to 168,
that the organopolysiloxanes (2) contain Si-bonded hydrogen in amounts of 0.011% to 0.044% by weight, preferably of 0.019% to 0.044% by weight, more preferably of 0.022% to 0.032% by weight,
and that the average number of Si-H groups per molecule is greater than 2 and less than 5,
or mixtures of (2) Si-H-functional organopolysiloxanes with
(2') Si-H-functional organopolysiloxanes of the general formula
H_{c}R_{3-c}SiO (R₂SiO)ₐ (RHSiO)_{b}SiR_{3-c}H_{c} (I')
where
c is 0 or 1, preferably 0,
R is as defined above,
a and b are integers, with the proviso that the sum of a+b is 8 to 248, preferably 38 to 248, and that the organopolysiloxanes (2') contain Si-bonded hydrogen in amounts of 0.045% to 0.35% by weight, preferably of 0.045% to 0.156% by weight,
in the presence of
(3) catalysts that promote the addition of Si-bonded hydrogen onto aliphatic multiple bonds,
where (1a), (1b) and (2) or mixtures of (2) and (2') are dispersed in
(4) diluents, preferably organopolysiloxanes having 2 to 200 silicon atoms or organic diluents or mixtures of organopolysiloxanes having 2 to 200 silicon atoms and organic diluents.

2. Organopolysiloxane gels according to Claim 1, **characterized in that** creamy, storage-stable organopolysiloxane gels are obtained after the reaction by subsequent homogenization, "storage-stable" meaning that the organopolysiloxane gels formed do not separate into two or more phases within 6 months of storage at room temperature and the softness of the gel does not change significantly within this period.

3. Organopolysiloxane gels according to Claim 2, **characterized in that** the organopolysiloxane gels thus obtained are diluted further by adding further diluents (4) and/or active ingredients for personal care or healthcare and optionally subsequent homogenization.

4. Organopolysiloxane gels according to Claim 1, 2 or 3, **characterized in that** the
(1a) unsaturated organopolysiloxane resins used are MQ resins composed of units of the formulae
SiO₂ (Q units)
and
R₃SiO_{1/2} and R₂R'SiO_{1/2} (M units),
where
R may be the same or different and is a monovalent, optionally substituted hydrocarbyl radical having 1 to 18 carbon atoms per radical,
R' is a monovalent hydrocarbyl radical onto which may be added Si-H groups in a hydrosilylation reaction, preferably an ω-alkenyl radical having 2 to 12 carbon atoms, preferably a vinyl radical, with the proviso that the MQ resins contain at least 2 R' radicals, preferably at least 3 R' radicals, and that the molar ratio of M units to Q units is in the range from 0.5 to 4.0, preferably in the range from 0.5 to 2.0, more preferably in the range from 0.6 to 1.5.

5. Organopolysiloxane gels according to any of Claims 1 to 4, **characterized in that** the compound (1b) used is one of the general formula
Z-(R¹O)ₚ-R² (III)
where
Z is a glycoside residue formed from 1 to 10 monosaccharide units, preferably glucose units,
R¹ may be the same or different and is a divalent hydrocarbyl radical having 2 to 4 carbon atoms,
R² is a monovalent hydrocarbyl radical having 2 to 12 carbon atoms, onto which may be added Si-H groups in a hydrosilylation reaction, preferably a monovalent hydrocarbyl radical having a terminal aliphatic multiple C-C bond and having 2 to 12 carbon atoms, preferably an allyl radical, and
p is 0 or an integer from 1 to 20.

6. Organopolysiloxane gels according to any of Claims 1 to 5, **characterized in that** the diluents (4) used are polydimethylsiloxanes having 2 to 50 silicon atoms, aliphatic or alicyclic hydrocarbons having 4 to 30 carbon atoms or esters of carboxylic acids having 2 to 30 carbon atoms.

7. Monophasic homogeneous mixtures comprising
(a) organopolysiloxane gels having glycoside residues according to any of Claims 1 to 6 and
(b) polar or hydrophilic solvents.

8. Mixtures according to Claim 7, **characterized in that** the polar or hydrophilic solvents (b) are those selected from the group of water, glycerol, ethylene glycol, diethylene glycol, propylene glycol and mixtures thereof.

9. Process for producing organopolysiloxane gels having glycoside residues according to any of Claims 1 to 6, **characterized in that**
(1a) unsaturated organopolysiloxane resins and
(1b) compounds having a glycoside residue and a hydrosilylatable end group,
with the proviso that the proportion by weight, based on the total weight of the organopolysiloxane gel, is 0.1% to 10% by weight, preferably 0.1% to 5% by weight, more preferably 0.3% to 2% by weight,
are reacted with
(2) Si-H-functional organopolysiloxanes of the general formula
H_{c}R_{3-c}SiO (R₂SiO)ₐ (RHSiO)_{b}SiR_{3-c}H_{c} (I)
where
c is 0 or 1, preferably 0,
R may be the same or different and is a monovalent, optionally substituted hydrocarbyl radical having 1 to 18 carbon atoms per radical, a and b are integers, with the proviso that the sum of a+b is 66 to 248, preferably 98 to 248, more preferably 118 to 168,
that the organopolysiloxanes (2) contain Si-bonded hydrogen in amounts of 0.011% to 0.044% by weight, preferably of 0.019% to 0.044% by weight, more preferably of 0.022% to 0.032% by weight,
and that the average number of Si-H groups per molecule is greater than 2,
or mixtures of (2) Si-H-functional organopolysiloxanes with
(2') Si-H-functional organopolysiloxanes of the general formula
H_{c}R_{3-c}SiO (R₂SiO)ₐ (RHSiO)_{b}SiR_{3-c}H_{c} (I')
where
c is 0 or 1, preferably 0,
R is as defined above,
a and b are integers, with the proviso that the sum of a+b is 8 to 248, preferably 38 to 248, and that the organopolysiloxanes (2') contain Si-bonded hydrogen in amounts of 0.045% to 0.35% by weight, preferably of 0.045% to 0.156% by weight,
in the presence of
(3) catalysts that promote the addition of Si-bonded hydrogen onto aliphatic multiple bonds,
where (1a), (1b) and (2) or mixtures of (2) and (2') are dispersed in
(4) diluents, preferably organopolysiloxanes having 2 to 200 silicon atoms or organic diluents or mixtures of organopolysiloxanes having 2 to 200 silicon atoms and organic diluents.

10. Process according to Claim 9, **characterized in that** the organopolysiloxane gels obtained after the reaction are homogenized, giving creamy, storage-stable organopolysiloxane gels, "storage-stable" meaning that the organopolysiloxane gels formed do not separate into two or more phases within 6 months of storage at room temperature and the softness of the gel does not change significantly within this period.

11. Process according to Claim 10, **characterized in that** the organopolysiloxane gels thus obtained are diluted with further diluents (4) and/or active ingredients for personal care or healthcare and then optionally homogenized.

12. Process according to Claim 9, 10 or 11, **characterized in that** the
(1a) unsaturated organopolysiloxane resins used are MQ resins composed of units of the formulae
SiO₂ (Q units)
and
R₃SiO_{1/2} and R₂R' SiO_{1/2} (M units),
where R and R' are each as defined in claim 4, with the proviso that the MQ resins contain at least 2 R' radicals, preferably at least 3 R' radicals, and that the molar ratio of M units to Q units is in the range from 0.5 to 4.0, preferably in the range from 0.5 to 2.0, more preferably in the range from 0.6 to 1.5.

13. Process according to any of Claims 9 to 12, **characterized in that** the compound (1b) used is one of the general formula
Z-(R¹O)ₚ-R² (III)
where Z, R¹, R² and p are each as defined in Claim 5.

14. Process according to any of Claims 9 to 13, **characterized in that** the diluents (4) used are polydimethylsiloxanes having 2 to 50 silicon atoms, aliphatic or alicyclic hydrocarbons having 4 to 30 carbon atoms or esters of carboxylic acids having 2 to 30 carbon atoms.

15. Cosmetic compositions comprising organopolysiloxane gels having glycoside residues according to any of Claims 1 to 6 or mixtures according to Claim 7 or 8 or
organopolysiloxane gels produced according to any of Claims 9 to 14.

## Revendications

1. Gels d'organopolysiloxane comprenant des radicaux glycoside, fabriqués par mise en réaction de
(la) des résines d'organopolysiloxane insaturées et
(1b) des composés qui comprennent un radical glycoside et un groupe terminal hydrosilylable, à condition que la proportion en poids, par rapport au poids total du gel d'organopolysiloxane, soit de 0,1 à 10 % en poids, de préférence de 0,1 à 5 % en poids, avantageusement de 0,3 à 2 % en poids,
avec
(2) des organopolysiloxanes à fonction Si-H de formule générale
H_{c}R_{3-c}SiO (R₂SiO)ₐ (RHSiO)_{b}SiR_{3-c}H_{c} (I),
dans laquelle
c représente 0 ou 1, de préférence 0,
les R peuvent être identiques ou différents, et signifient un radical hydrocarboné monovalent, éventuellement substitué, de 1 à 18 atomes de carbone par radical,
a et b sont des nombres entiers, à condition que la somme a+b soit de 66 à 248, de préférence de 98 à 248, avantageusement de 118 à 168,
que les organopolysiloxanes (2) contiennent de l'hydrogène relié à Si en quantités de 0,011 à 0,044 % en poids, de préférence de 0,019 à 0,044 % en poids, avantageusement de 0,022 à 0,032 % en poids,
et que le nombre de groupes Si-H par molécule soit en moyenne supérieur à 2 et inférieur à 5,
ou des mélanges de (2) des organopolysiloxanes à fonction Si-H avec
(2') des organopolysiloxanes à fonction Si-H de formule générale
H_{c}R_{3-c}SiO(R₂SiO)ₐ(RHSiO)_{b}SiR_{3-c}H_{c} (I'),
dans laquelle
c représente 0 ou 1, de préférence 0,
les R ont la signification indiquée précédemment,
a et b sont des nombres entiers, à condition que la somme a+b soit de 8 à 248, de préférence de 38 à 248,
et que les organopolysiloxanes (2') contiennent de l'hydrogène relié à Si en quantités de 0,045 à 0,35 % en poids, de préférence de 0,045 à 0,156 % en poids,
en présence de
(3) des catalyseurs favorisant l'addition d'hydrogène relié à Si sur une liaison multiple aliphatique,
(la), (1b) et (2) ou les mélanges de (2) et (2') étant dispersés dans
(4) des diluants, de préférence des organopolysiloxanes contenant 2 à 200 atomes Si ou des diluants organiques ou des mélanges d'organopolysiloxanes contenant 2 à 200 atomes Si et de diluants organiques.

2. Gels d'organopolysiloxane selon la revendication 1, **caractérisés en ce qu'**après la réaction, des gels d'organopolysiloxane crémeux, stables au stockage, sont obtenus par homogénéisation ultérieure, stables au stockage signifiant que les gels d'organopolysiloxane formés ne se séparent pas en deux phases ou plus pendant 6 mois de stockage à température ambiante et que la blancheur du gel n'est essentiellement pas modifiée pendant cette durée.

3. Gels d'organopolysiloxane selon la revendication 2, **caractérisés en ce que** les gels d'organopolysiloxane ainsi obtenus sont davantage dilués par ajout de diluants supplémentaires (4) et/ou d'agents actifs pour le soin du corps ou l'hygiène et éventuellement homogénéisation ultérieure.

4. Gels d'organopolysiloxane selon la revendication 1, 2 ou 3, **caractérisés en ce qu'**en tant que
(la) résines d'organopolysiloxane insaturées,
des résines MQ constituées d'unités des formules
SiO₂ (unités Q)
et
R₃SiO_{1/2} et R₂R'SiO_{1/2} (unités M),
sont utilisées,
dans lesquelles
les R peuvent être identiques ou différents et signifient un radical hydrocarboné monovalent, éventuellement substitué, de 1 à 18 atomes de carbone par radical,
R' signifie un radical hydrocarboné monovalent, sur lequel des groupes Si-H peuvent être additionnés par une réaction d'hydrosilylation, de préférence un radical ω-alcényle de 2 à 12 atomes C, avantageusement un radical vinyle,
à condition que les résines MQ contiennent au moins 2 radicaux R', de préférence au moins 3 radicaux R', et que le rapport molaire entre les unités M et les unités Q se situe dans la plage allant de 0,5 à 4,0, de préférence dans la plage allant de 0,5 à 2,0, avantageusement dans la plage allant de 0,6 à 1,5.

5. Gels d'organopolysiloxane selon l'une quelconque des revendications 1 à 4, **caractérisés en ce qu'**en tant que composé (1b), des composés de formule générale
Z-(R¹O)ₚ-R² (III),
sont utilisés,
dans laquelle
Z signifie un radical glycoside qui est formé par 1 à 10 unités monosaccharide, de préférence unités glucose,
les R¹ peuvent être identiques ou différents, et signifient un radical hydrocarboné bivalent de 2 à 4 atomes de carbone,
R² signifie un radical hydrocarboné monovalent de 2 à 12 atomes C, sur lequel des groupes Si-H peuvent être additionnés par une réaction d'hydrosilylation, de préférence un radical hydrocarboné monovalent comprenant une liaison multiple C-C aliphatique terminale, de 2 à 12 atomes C, avantageusement un radical allyle, et
p signifie 0 ou un nombre entier de 1 à 20.

6. Gels d'organopolysiloxane selon l'une quelconque des revendications 1 à 5, **caractérisés en ce qu'**en tant que diluants (4), des polydiméthylsiloxanes de 2 à 50 atomes Si, des hydrocarbures aliphatiques ou alicycliques de 4 à 30 atomes C ou des esters d'acides carboxyliques de 2 à 30 atomes C sont utilisés.

7. Mélanges homogènes monophasés contenant :
(a) des gels d'organopolysiloxane comprenant des radicaux glycoside selon l'une quelconque des revendications 1 à 6, et
(b) des solvants polaires ou hydrophiles.

8. Mélanges selon la revendication 7, **caractérisés en ce que** les solvants polaires ou hydrophiles (b) sont choisis dans le groupe constitué par l'eau, la glycérine, l'éthylène glycol, le diéthylène glycol, le propylène glycol et leurs mélanges.

9. Procédé de fabrication des gels d'organopolysiloxane comprenant des radicaux glycoside selon l'une quelconque des revendications 1 à 6, **caractérisé en ce que**
(la) des résines d'organopolysiloxane insaturées et
(1b) des composés qui comprennent un radical glycoside et un groupe terminal hydrosilylable,
à condition que la proportion en poids, par rapport au poids total du gel d'organopolysiloxane, soit de 0,1 à 10 % en poids, de préférence de 0,1 à 5 % en poids, avantageusement de 0,3 à 2 % en poids,
sont mis en réaction avec
(2) des organopolysiloxanes à fonction Si-H de formule générale
H_{c}R_{3-c}SiO(R₂SiO)ₐ (RHSiO)_{b}SiR_{3-c}H_{c} (I),
dans laquelle
c représente 0 ou 1, de préférence 0,
les R peuvent être identiques ou différents, et signifient un radical hydrocarboné monovalent, éventuellement substitué, de 1 à 18 atomes de carbone par radical,
a et b sont des nombres entiers, à condition que la somme a+b soit de 66 à 248, de préférence de 98 à 248, avantageusement de 118 à 168,
que les organopolysiloxanes (2) contiennent de l'hydrogène relié à Si en quantités de 0,011 à 0,044 % en poids, de préférence de 0,019 à 0,044 % en poids, avantageusement de 0,022 à 0,032 % en poids,
et que le nombre de groupes Si-H par molécule soit en moyenne supérieur à 2,
ou des mélanges de (2) des organopolysiloxanes à fonction Si-H avec
(2') des organopolysiloxanes à fonction Si-H de formule générale
H_{c}R_{3-c}SiO (R₂SiO)ₐ (RHSiO)_{b}SiR_{3-c}H_{c} (I'),
dans laquelle
c représente 0 ou 1, de préférence 0,
les R ont la signification indiquée précédemment,
a et b sont des nombres entiers, à condition que la somme a+b soit de 8 à 248, de préférence de 38 à 248,
et que les organopolysiloxanes (2') contiennent de l'hydrogène relié à Si en quantités de 0,045 à 0,35 % en poids, de préférence de 0,045 à 0,156 % en poids,
en présence de
(3) des catalyseurs favorisant l'addition d'hydrogène relié à Si sur une liaison multiple aliphatique,
(la), (1b) et (2) ou les mélanges de (2) et (2') étant dispersés dans
(4) des diluants, de préférence des organopolysiloxanes contenant 2 à 200 atomes Si ou des diluants organiques ou des mélanges d'organopolysiloxanes contenant 2 à 200 atomes Si et de diluants organiques.

10. Procédé selon la revendication 9, **caractérisé en ce que** les gels d'organopolysiloxane obtenus après la réaction sont homogénéisés, des gels d'organopolysiloxane crémeux, stables au stockage, étant obtenus, stables au stockage signifiant que les gels d'organopolysiloxane formés ne se séparent pas en deux phases ou plus pendant 6 mois de stockage à température ambiante et que la blancheur du gel n'est essentiellement pas modifiée pendant cette durée.

11. Procédé selon la revendication 10, **caractérisé en ce que** les gels d'organopolysiloxane ainsi obtenus sont davantage dilués par ajout de diluants supplémentaires (4) et/ou d'agents actifs pour le soin du corps ou l'hygiène, puis éventuellement homogénéisés.

12. Procédé selon la revendication 9, 10 ou 11, **caractérisé en ce qu'**en tant que (la) résines d'organopolysiloxane insaturées,
des résines MQ constituées d'unités des formules
SiO₂ (unités Q)
et
R₃SiO_{1/2} et R₂R'SiO_{1/2} (unités M),
sont utilisées,
dans lesquelles R et R' ont la signification indiquée dans la revendication 4,
à condition que les résines MQ contiennent au moins 2 radicaux R', de préférence au moins 3 radicaux R', et que le rapport molaire entre les unités M et les unités Q se situe dans la plage allant de 0,5 à 4,0, de préférence dans la plage allant de 0,5 à 2,0, avantageusement dans la plage allant de 0,6 à 1,5.

13. Procédé selon l'une quelconque des revendications 9 à 12, **caractérisés en ce qu'**en tant que composé (1b), des composés de formule générale
Z-(R¹O)ₚ-R² (III),
sont utilisés,
dans laquelle Z, R¹, R² et p ont la signification indiquée dans la revendication 5.

14. Procédé selon l'une quelconque des revendications 9 à 13, **caractérisés en ce qu'**en tant que diluants (4), des polydiméthylsiloxanes de 2 à 50 atomes Si, des hydrocarbures aliphatiques ou alicycliques de 4 à 30 atomes C ou des esters d'acides carboxyliques de 2 à 30 atomes C sont utilisés.

15. Compositions cosmétiques contenant des gels d'organopolysiloxane comprenant des radicaux glycoside selon l'une quelconque des revendications 1 à 6 ou des mélanges selon la revendication 7 ou 8 ou des gels d'organopolysiloxane fabriqués selon l'une quelconque des revendications 9 à 14.
